# EUROPEAN PATENT SPECIFICATION

(11) **EP 1 090 026 B1**
(45) Date of publication and mention of the grant of the patent: **27.08.2008**
(21) Application number: 99930624.4
(22) Date of filing: 23.06.1999
(51) Int. Cl.: C12N 15/54, A01K 67/027, A61P 3/04

(54) **DIACYLGLYCEROL O-ACYLTRANSFERASE**
DIACYLGLYCERIN O-ACYLTRANSFERASE
DIACYLGLYCEROL O-ACYLTRANSFERASE

(30) Priority: 24.06.1998 US 103754; 09.11.1998 US 107771 P
(43) Date of publication of application: 11.04.2001
(73) Proprietor: The J. David Gladstone Institutes, San Francisco, CA 94158 (US); United States Government Represented by the Department of Veterans Affairs, Washington DC 20420 (US); The Regents of the University of California, Oakland, CA 94607-5200 (US)
(72) Inventor: FARESE, Robert V. Jr. Gladstone Institute, San Francisco, CA 94141-9100 (US); CASES, Sylvaine Gladstone Institute, San Francisco, CA 94141-9100 (US); SMITH, Steven Gladstone Institute, San Francisco, CA 94141-9100 (US); ERICKSON, Sandra K. Veteran's Adm. Med. Center, San Francisco, CA 94121 (US); CHEN, Hubert, San diego, CA 92130 (US)
(74) Representative: Brasnett, Adrian Hugh
(86) International application number: PCT/US1999/014245
(87) International publication number: WO 1999/067268

(56) References cited:
- KATAVIC V ET AL: "ALTERATION OF SEED FATTY ACID COMPOSITION BY AN ETHYL METHANESULFONATE-INDUCED MUTATION IN ARABIDOPSIS THALIANA AFFECTING DIACYLGLYCEROL ACYLTRANSFERASE ACTIVITY" PLANT PHYSIOLOGY, AMERICAN SOCIETY OF PLANT PHYSIOLOGISTS, ROCKVILLE, MD, US, vol. 108, 1995, pages 399-409, XP002915657 ISSN: 0032-0889
- CASES S ET AL: "CLONING AND EXPRESSION OF A CANDIDATE GENE FOR DIACYLGLYCEROL ACYLTRANSFERASE" FASEB JOURNAL, FED. OF AMERICAN SOC. FOR EXPERIMENTAL BIOLOGY, BETHESDA, MD, US, vol. 12, no. 5, 20 March 1998 (1998-03-20), page A814 XP002915658 ISSN: 0892-6638
- FARESE FV JR.: "Acyl CoA:cholesterol acyltransferase genes and knockout mice" CURRENT OPINION IN LIPIDOLOGY, vol. 9, no. 2, April 1998 (1998-04), pages 119-123, XP008026803 ISSN: 0957-9672
- FRENTZEN M (REPRINT): "Acyltransferases from basic science to modified seed oils" FETT - LIPID, WILEY-VCH VERLAG,WEINHEIM, DE, vol. 100, no. 4/5, May 1998 (1998-05), pages 161-166, XP002122744 ISSN: 0931-5985
- WILSON R F ET AL: "RECENT DEVELOPMENTS IN THE MOLECULAR BIOCHEMISTRY AND GENETICS OF DIACYGLYCEROL ACYLTRANSFERASE FROM SOYBEAN" SEED OILS FOR THE FUTURE, AOCS PRESS, CHAMPAIGN, IL, US, 1992, pages 116-135, XP000884791
- TABATA N ET AL: "Xanthohumols, diacylglycerol acyltransferase inhibitors, from Humulus Lupulus" PHYTOCHEMISTRY, PERGAMON PRESS, GB, vol. 46, no. 4, October 1997 (1997-10), pages 683-687, XP004293462 ISSN: 0031-9422
- ZOU ET AL: "The Arabidopsis thaliana TAG1 mutant has a mutation in a diacylglycerol acyltransferase gene" PLANT JOURNAL, BLACKWELL SCIENTIFIC PUBLICATIONS, OXFORD, GB, vol. 19, no. 6, September 1999 (1999-09), pages 645-653, XP002133607 ISSN: 0960-7412
- SMITH S J ET AL: "Obesity resistance and multiple mechanisms of triglyceride synthesis in mice lacking Dgat" NATURE GENETICS, NATURE AMERICA, NEW YORK, US, vol. 25, no. 1, May 2000 (2000-05), pages 87-90, XP002221994 ISSN: 1061-4036
- CHEN HC ET AL: "Increased insulin and leptin sensitivity in mice lacking acyl CoA:diacylglycerol acyltransferase I" THE JOURNAL OF CLINICAL INVESTIGATION, vol. 109, no. 8, April 2002 (2002-04), pages 1049-1055, XP001184178
- BUHMAN KIMBERLY K ET AL: "DGAT1 is not essential for intestinal triacylglycerol absorption or chylomicron synthesis." THE JOURNAL OF BIOLOGICAL CHEMISTRY. UNITED STATES 12 JUL 2002, vol. 277, no. 28, 12 July 2002 (2002-07-12), pages 25474-25479, XP002272033 ISSN: 0021-9258
- ANDERSSON M ET AL: "PURIFICATION OF DIACYLGLYCEROL: ACYLTRANSFERASE FROM RAT LIVER TO NEAR HOMOGENEITY" JOURNAL OF LIPID RESEARCH, BETHESDA, MD, US, vol. 35, 1994, pages 535-545, XP002915656 ISSN: 0022-2275
- CASES et al., "Identification of a Gene Encoding an Acyl CoA:Diacylglycerol Acyltransferase, a Key Enzyme in Triacylglycerol Synthesis", PROC. NATL. ACAD. SCI. USA, October 1998, Vol. 95, pages 13018-13023, XP002919933
- CASES et al., "Cloning and Expression of a Candidate Gene for Diacylglycerol Acyltransferase", FASEB J., 20 March 1998, Vol. 12, No. 5, page A814, Abstract Number 4718, XP002919934
- OELKERS P. ET AL: 'Characterization of two human genes encoding acyl coenzyme A:cholesterol acyltransferase-related enzymes.' JOURNAL OF BIOLOGICAL CHEMISTRY vol. 273, no. 41, October 1998, pages 26765 - 26771, XP002122746
- DATABASE GENBANK [Online] 15 October 1998 OELKERS P. ET AL: 'Homo sapiens ACAT related gene product 1 mRNA, complete cds.' Database accession no. AF059202
- DATABASE GENBANK [Online] 19 September 1997 NEWMAN T. ET AL: '24635 CD4-13 Arabidopsis thaliana cDNA clone E6B2T7, mRNA sequence.' Database accession no. AA042298

## Description

### INTRODUCTION

### Field of the Invention

The field of the invention is enzymes, particularly acyltransferases.

### Background of the Invention

Diacylglycerol O-Acyltransferase (EC 2.3.1.20), also known as diglyceride acyltransferase or DGAT, is a critical enzyme in triacylglycerol synthesis. Triacylglycerols are quantitatively the most important storage form of energy for eukaryotic cells. DGAT catalyzes the rate-limiting and terminal step in triacylglycerol synthesis using diacylglycerol and fatty acyl CoA as substrates. As such, DGAT plays a fundamental role in the metabolism of cellular diacylglycerol and is important in higher eukaryotes for intestinal fat absorption, lipoprotein assembly, fat storage in adipocytes, milk production and possibly egg production and sperm maturation.

Because of its central role in a variety of different processes, there is much interest in the identification of polynucleotides encoding proteins having DGAT activity, as well as the proteins encoded thereby.

### Relevant Literature

References describing at least partial purification of DGAT from naturally occurring sources include: Kamisaka et al., "Purification and Characterization of Diacylglycerol Acyltransferase from the Lipid Body Fraction of Oleaginous Fungus," J. Biochem (Tokyo) 1997 (6) 1107-1114; Little et al., "Solubilization and Characterization of Diacylglycerol Acyltransferase from Microspore-Derived Cultures of Oilseed Rape," Biochem J. (December 15, 1994) 304 (Pt 3): 951-958; Andersson et al., "Purification of Diacylglycerol:acyltransferase from Rat Liver to Near Homogeneity," J. Lipid Res. (March 1994) 35: 535-545; Polokoff & Bell, "Solubilization, Partial Purification and Characterization of Rat Liver Microsomal Diacylglycerol Acyltransferase," Biochim. Biophys. Acta (1980) 618: 129-142.

Also of interest are: Cases et al., "Identification of a gene encoding an acyl CoA:diacylglycerol acyltransferase, a key enzyme in triacylglycerol synthesis," Proc. Natl. Acad. Sci. USA (October 1998) 95:13018-13023; and Oelkers et al., "Characterization of Two Human Genes Encoding Acyl Coenzyme A: Cholesterol Acyltransferase-Related Enzymes," J. Biol. Chem. (October 9, 1998) 273:26765-71.

References describing the role DGAT plays in various biological processes include: Bell & Coleman, "Enzymes of Glycerolipid Synthesis in Eukaryotes," Annu. Rev. Biochem. (1980) 49: 459-487; Lehner & Kuksis, "Biosynthesis of Triacylglycerols," Prog. Lipid Res. (1996) 35: 169-201; Brindley, Biochemistry of Lipids, Lipoproteins and Membranes (eds. Vance & Vance)(Elsevier, Amsterdam)(1991) pp171-203; Haagsman & Van Golde, "Synthesis and Secretion of Very Low Density Lipoproteins by Isolated Rat Hepatocytes in Suspension: Role of Diacylglycerol Acyltransferase," Arch. Biochem. Biophys. (1981) 208:395-402; Coleman & Bell, "Triacylglycerol Synthesis in Isolated Fat Cells. Studies on the Microsomal Diacylglycerol Acyltransferase Activity Using Ethanol-Dispersed Diacylglycerols". J. Biol. Chem. (1976) 251:4537-4543.

Cases et al., "Cloning and Expression of a Candidate Gene for Diacylglycerol Acyltransferase" FASEB Journal, Fed. Of American Soc. For Experimental Biology, Bethesda, MD, US, vol 12, No. 5, mach 1998, page A 814, XP002915658, ISSN: 0892-6638 describes the cloning of a cDNA, designated A-3, which encodes a polypeptide having DGAT activity which is 20% identical to acyl CoA:cholesterol acyltransferase (ACAT).

### SUMMARY OF THE INVENTION

Nucleic acid compositions encoding polypeptide products with diglyceride acyltransferase activity, as well as the polypeptide products encoded thereby and methods for producing the same, are disclosed. Also disclosed are: methods and compositions for modulating DGAT activity; DGAT transgenic cells, animals and plants, as well as methods for their preparation; and methods for making triglycerides and triglyceride compositions, as well as the compositions produced by these methods. The subject methods and compositions find use in a variety of different applications, including research, medicine, agriculture and industry.

### BRIEF DESCRIPTION OF THE FIGURES

Fig. 1 depicts the strategy employed to generate the DGAT knockout mice of the subject invention.
Fig. 2 shows the DGAT activity in various tissue membranes of DGAT knockout and wild type mice.
Fig. 3 compares the fat pads of wild type and DGAT knockout mice.
Fig. 4 compares the body weights of wild-type and DGAT knockout mice fed a chow diet.
Fig. 5 compares the body weights of wild type and DGAT knockout mice fed a high fat diet.
Figs. 6A and 6B compare the body weight and fat pads of 30 week old female wild type and DGAT knockout mice fed a high fat diet.
Fig. 7 compares the glucose and free fatty acid levels in wild type and DGAT knockout mice.

### DETAILED DESCRIPTION OF THE INVENTION

Nucleic acid compositions encoding polypeptide products with diglyceride acyltransferase activity, as well as the polypeptide products encoded thereby and methods for producing the same, are disclosed. Also disclosed are: methods and compositions for modulating DGAT activity, e.g. in the treatment of disease conditions associated with DGAT activity, including obesity; DGAT transgenic cells, animals, plants and fungi, and methods for their preparation, e.g. for use in research, food production, industrial feedstock production, etc.; and methods for making triglycerides and triglyceride compositions, e.g. oils. The methods and compositions of the subject invention find use in a variety of different applications and fields, including research, medicine, agriculture and industry.

The present invention provides a non-human transgenic mammal, wherein the non-human transgenic mammal comprises a diacylglycerol *O*-acyltransferase (DGAT) transgene encoding an enzymatically active DGAT polypeptide, and wherein the DGAT transgene comprises a nucleotide sequence having at least 75% nucleotide sequence identity to the nucleotide sequence set forth in SEQ ID NO:3. In one embodiment, the mammal is a rat or a mouse.

In another embodiment the transgene is a DGAT transgene comprising a nucleotide sequence having at least 90% nucleotide sequence identity, preferably at least 95% nucleotide sequence identity, to the nucleotide sequence set forth in SEQID NO:3.

In a further embodiment, the DGAT transgene comprises a non-endogenous nucleic acid. The invention further provides a transgenic cell derived from said non-human transgenic mammal.

The invention further provides a non-human transgenic knock-out mammal, wherein the non-human transgenic knock-out mammal comprises a disrupted endogenous diacyglycerol *O*-acyltransferase (DGAT) locus, and wherein said knock-out mammal has reduced adipose tissue compared to a non-transgenic mammal of the same species. In one embodiment, the mammal is a rat or a mouse. In another embodiment, the knock-out mammal is leaner than a non-transgenic mammal of the same species. In a further embodiment, the fat cells in said knock-out mammal are smaller than fat cells in a non-transgenic mammal of the same species.

The invention further provides a transgenic cell derived from said non-human transgenic knock-out mammal .The invention further provides a method of screening a test candidate for its ability to modulate diacylglycerol *O-*acyltransferase (DGAT) activity, said method comprising:
contacting a mammal or a cell of the invention with said test candidate; and
determining the effect of said test candidate on DGAT activity in said mammal or cell.

Before the subject invention is further described, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an," and "the" include plural reference unless the context clearly dictates otherwise. Unless defined otherwise, all technical and scientific terms used herein have the same meaning as commonly understood to one of ordinary skill in the art to which this invention belongs.

### NUCLEIC ACID COMPOSITIONS

Nucleic acid compositions encoding polypeptide products, as well as fragments thereof, having diglyceride acetyltransferase (DGAT) activity are disclosed. By nucleic acid composition is meant a composition comprising a sequence of DNA having an open reading frame that encodes a DGAT polypeptide, i.e. a gene encoding a polypeptide having DGAT activity, and is capable, under appropriate conditions, of being expressed as a DGAT polypeptide. Also encompassed in this term are nucleic acids that are homologous or substantially similar or identical to the nucleic acids encoding DGAT polypeptides or proteins. Thus, disclosed are genes encoding mammalian DGAT, such as genes encoding human DGAT and homologs thereof and mouse DGAT and homologs thereof, as well as plant DGAT, such as Arabidopsis thaliana DGAT and homologs thereof. In other words, both animal and plant genes encoding DGAT proteins are disclosed. Non-human nucleic acids encoding DGAT products are also disclosed. The coding sequence of the human *DGAT* gene, i.e. the human cDNA encoding the human DGAT enzyme, includes or comprises a nucleic acid sequence substantially the same as or identical to that identified as SEQ ID NO:01 or SEQ ID NO:02, *infra.* A nucleic acid sequence encoding the full length human DGAT enzyme (i.e. a protein having or including the sequence of SEQ ID NO:05) is also of interest. Also of interest is a nucleic acid encoding the full length sequence of the human DGAT protein, as shown in SEQ ID NO:06. The coding sequence of the mouse DGAT gene, i.e. the mouse cDNA encoding the mouse DGAT enzyme, has the nucleic acid sequence identified as SEQ ID NO:03, *infra.* Also of interest is the sequence identified as SEQ ID NO:09. The coding sequence of the A. thaliana DGAT gene, i.e. the A.thaliana cDNA encoding the A. thaliana DGAT enzyme, comprises or includes the nucleic acid sequence identified as SEQ ID NO:04, *infra.*

The source of homologous genes to those specifically listed above may be any species, including both animal and plant species, e.g., primate species, particularly human; rodents, such as rats and mice, canines, felines, bovines, ovines, equines, yeast, nematodes, etc. Between mammalian species, *e.g*., human and mouse, homologs have substantial sequence similarity, *e.g.* at least 75% sequence identity, usually at least 90%, more usually at least 95% between nucleotide sequences. Sequence similarity is calculated based on a reference sequence, which may be a subset of a larger sequence, such as a conserved motif, coding region, flanking region, *etc.* A reference sequence will usually be at least about 18 nt long, more usually at least about 30 nt long, and may extend to the complete sequence that is being compared. Algorithms for sequence analysis are known in the art, such as BLAST, described in Altschul et al. (1990), J. Mol. Biol. 215:403-10. Unless specified otherwise, all sequence identity values provided herein are determined using GCG (Genetics Computer Group, Wisconsin Package, Standard Settings, gap creation penalty 3.0, gap extension penalty 0.1). The sequences provided herein are essential for recognizing DGAT- related and homologous polynucleotides in database searches.

Nucleic acids encoding the DGAT proteins and DGAT polypeptides may be cDNAs or genomic DNAs, as well as fragments thereof. The term "DGAT- gene" shall be intended to mean the open reading frame encoding specific DGAT proteins and polypeptides, and DGAT introns, as well as adjacent 5' and 3' non-coding nucleotide sequences involved in the regulation of expression, up to about 20 kb beyond the coding region, but possibly further in either direction. The gene may be introduced into an appropriate vector for extrachromosomal maintenance or for integration into a host genome.

The term "cDNA" as used herein is intended to include all nucleic acids that share the arrangement of sequence elements found in native mature mRNA species, where sequence elements are exons and 3' and 5' non-coding regions. Normally mRNA species have contiguous exons, with the intervening introns, when present, being removed by nuclear RNA splicing, to create a continuous open reading frame encoding a DGAT protein.

A genomic sequence of interest comprises the nucleic acid present between the initiation codon and the stop codon, as defined in the listed sequences, including all of the introns that are normally present in a native chromosome. It may further include the 3' and 5' untranslated regions found in the mature mRNA. It may further include specific transcriptional and translational regulatory sequences, such as promoters, enhancers, etc., including about 1 kb, but possibly more, of flanking genomic DNA at either the 5' or 3' end of the transcribed region. The genomic DNA may be isolated as a fragment of 100 kbp or smaller; and substantially free of flanking chromosomal sequence. The genomic DNA flanking the coding region, either 3' or 5', or internal regulatory sequences as sometimes found in introns, contains sequences required for proper tissue and stage specific expression.

The nucleic acid compositions may encode all or a part of the subject DGAT proteins and polypeptides, described in greater detail *infra.* Double or single stranded fragments may be obtained from the DNA sequence by chemically synthesizing oligonucleotides in accordance with conventional methods, by restriction enzyme digestion, by PCR amplification, *etc*. For the most part, DNA fragments will be of at least 15 nt, usually at least 18 nt or 25 nt, and may be at least about 50 nt.

The DGAT- genes are isolated and obtained in substantial purity, generally as other than an intact chromosome. Usually, the DNA will be obtained substantially free of other nucleic acid sequences that do not include a DGAT sequence or fragment thereof, generally being at least about 50%, usually at least about 90% pure and are typically "recombinant", *i.e.* flanked by one or more nucleotides with which it is not normally associated on a naturally occurring chromosome.

In addition to the plurality of uses described in greater detail in following sections, the subject nucleic acid compositions find use in the preparation of all or a portion of the DGAT polypeptides, as described below.

### POLYPEPTIDE COMPOSITIONS

Also disclosed are polypeptides having DGAT activity, i.e. capable of catalyzing the acylation of diacylglycerol. In addition to being capable of catalyzing the esterificationof diacylglycerol with a fatty acyl CoA substrates, the subject proteins are incapable of esterifying, at least to any substantial extent, the following substrates: cholesterol, 25-hydroxy-, 27-hydroxy-,7α-hydroxy- or 7β-hydroxycholesterols, 7-ketocholesterol, vitamins D2 and D3, vitamin E, dehydrepiandrosterone, retinol, ethanol, sitosterol, lanosterol and ergosterol.

The term polyeptide composition as used herein refers to both the full length proteins as well as portions or fragments thereof. Also included in this term are variations of the naturally occurring proteins, where such variations are homologous or substantially similar to the naturally occurring protein, as described in greater detail below, be the naturally occurring protein the human protein, mouse protein, or protein from some other species which naturally expresses a DGAT enzyme, be that species animal or plant. In the following description of the subject invention, the term DGAT is used to refer not only to the human form of the enzyme, but also to homologs thereof expressed in non-human species, including plant species.

The subject DGAT proteins are, in their natural environment, trans-membrane proteins. The subject proteins are characterized by the presence of at least one potential N-linked glycosylation site, at least one potential tyrosine phosphorylation site, and multiple hydrophobic domains, including 6 to 12 hydrophobic domains capable of serving as trans-membrane regions. The proteins range in length from about 400 to 650, usually from about 475 to 525 and more usually from about 485 to 500 amino acid residues, and the projected molecular weight of the subject proteins based solely on the number of amino acid residues in the protein ranges from about 50 to 80, usually from about 55 to 75 and more usually from about 60 to 65 kDa, where the actual molecular weight may vary depending on the amount of glycolsylation of the protein and the apparent molecular weight may be considerably less (e.g. 40 to 50 kDa) because of SDS binding on gels.

The amino acid sequences of the subject proteins are characterized by having at least some homology to a corresponding ACAT protein from the same species, e.g. a human DGAT protein has at least some sequence homology with the human ACAT-1 protein, the mouse DGAT protein has at least some sequence homology with the mouse ACAT-1 protein, etc., where the sequence homology will not exceed about 50%, and usually will not exceed about 40% and more usually will not exceed about 25%, but will be at least about 15% and more usually at least about 20%, as determined using GCG (Genetics Computer Group, Wisconsin Package, Standard Settings, Gap Creation Penalty 3.0, Gap Extension Penalty 0.1).

Of particular interest are proteins that are non-naturally glycosylated. By non-naturally glycosylated is meant that the protein has a glycosylation pattern, if present, which is not the same as the glycosylation pattern found in the corresponding naturally occurring protein. For example, human DGAT is characterized by having a glycosylation pattern, if it is glycosylated at all, that differs from that of naturally occurring human DGAT. Thus, the non-naturally glycosylated DGAT proteins include non-glycosylated DGAT proteins, i.e. proteins having no covalently bound glycosyl groups.

Of particular interest is the human DGATprotein, where the human DGAT protein has an amino acid sequence that comprises or includes a region substantially the same as or identical to the sequence appearing as SEQ ID NO:05 *infra.* The sequence of the full length human DGAT protein includes the sequence provided in SEQ ID NO:06 *infra.* As such, DGAT proteins having an amino acid sequence that is substantially the same as or identical to the sequence of SEQ ID NO:6 are of interest. By substantially the same as is meant a protein having a region with a sequence that has at least about 75%, usually at least about 90% and more usually at least about 98 % sequence identity with the sequence of SED ID NO:06, as measured by GCG, supra. Of particular interest is the mouse DGAT protein, where the mouse DGAT protein has an amino acid sequence that is substantially the same as or identical to the sequence appearing as SEQ ID NO:07, infra. Also of interest is SEQ ID NO:10. Also of particular interest is the A. thaliana DGAT protein, where the A. thaliana DGAT protein has an amino acid sequence encoded by the nucleic acid comprising the sequence appearing as SEQ ID NO:04, *infra.*

In addition to the specific DGAT proteins described above, homologs or proteins (or fragments thereof) from other species, i.e. other animal or plant species, are also disclosed, where such homologs or proteins may be from a variety of different types of species, including animals, such as mammals, e.g. rodents, such as mice, rats; domestic animals, e.g. horse, cow, dog, cat; and humans, as well as non-mammalian species, e.g. avian, insect and the like, as well plant species. By homolog is meant a protein having at least about 35 %, usually at least about 40% and more usually at least about 60 % amino acid sequence identity the specific DGAT proteins as identified in SEQ ID NOS: 04 to 06, where sequence identity is determined using GCG, *supra.*

The DGAT proteins (e.g. human DGAT or a homolog thereof; non-human DGAT proteins, e.g. mouse DGAT, plant DGAT) are present in a non-naturally occurring environment, *e.g.* are separated from their naturally occurring environment. In certain embodiments, the subject DGAT is present in a composition that is enriched for DGAT as compared to DGAT in its naturally occurring environment. As such, purified DGAT is provided, where by purified is meant that DGAT is present in a composition that is substantially free of non DGAT proteins, where by substantially free is meant that less than 90 %, usually less than 60 % and more usually less than 50 % of the composition is made up of non-DGAT proteins. For compositions that are enriched for DGAT proteins, such compositions will exhibit a DGAT activity of at least about 100, usually at least about 200 and more usually at least about 1000 pmol triglycerides formed/mg protein/min, where such activity is determined by the assay described in the Experimental Section, infra.

The DGAT protein may be present in a composition that is substantially free of the constituents that are present in its naturally occurring environment. For example, a human DGAT protein comprising composition will be substantially, if not completely, free of those other biological constituents, such as proteins, carbohydrates, lipids, etc., with which it is present in its natural environment. As such, these protein compositions will necessarily differ from those that are prepared by purifying the protein from a naturally occurring source, where at least trace amounts of the protein's constituents will still be present in the composition prepared from the naturally occurring source.

The DGAT may also be present as an isolate, by which is meant that the DGAT is substantially free of both non-DGAT proteins and other naturally occurring biologic molecules, such as oligosaccharides, polynucleotides and fragments thereof, and the like, where substantially free in this instance means that less than 70 %, usually less than 60% and more usually less than 50 % of the composition containing the isolated DGAT is a non-DGAT naturally occurring biological molecule. The DGAT may be present in substantially pure form, where by substantially pure form is meant at least 95%, usually at least 97% and more usually at least 99% pure.

In addition to the naturally occurring DGAT proteins, DGAT polypeptides which vary from the naturally occurring DGAT proteins are also disclosed By DGAT polypeptides is meant proteins having an amino acid sequence encoded by an open reading frame (ORF) of a DGAT gene, described *supra,* including the full length DGAT protein and fragments thereof, particularly biologically active fragments and/or fragments corresponding to functional domains; and including fusions of the subject polypeptides to other proteins or parts thereof. Fragments of interest will typically be at least about 10 aa in length, usually at least about 50 aa in length, and may be as long as 300 aa in length or longer, but will usually not exceed about 1000 aa in length, where the fragment will have a stretch of amino acids that is identical to a DGAT protein of SEQ ID NO:5, SEQ ID NO:06, SEQ ID NO:07 or a homolog thereof; of at least about 10 aa, and usually at least about 15 aa, and in many embodiments at least about 50 aa in length.

### PREPARATION OF DGAT POLYPEPTIDES

The subject DGAT proteins and polypeptides may be obtained from naturally occurring sources, but are preferably synthetically produced. Where obtained from naturally occurring sources, the source chosen will generally depend on the species from which the DGAT is to be derived.

The subject DGAT polypeptide compositions may be synthetically derived by expressing a recombinant gene encoding DGAT, such as the polynucleotide compositions described above, in a suitable host. For expression, an expression cassette may be employed. The expression vector will provide a transcriptional and translational initiation region, which may be inducible or constitutive, where the coding region is operably linked under the transcriptional control of the transcriptional initiation region, and a transcriptional and translational termination region. These control regions may be native to a DGAT gene, or may be derived from exogenous sources.

Expression vectors generally have convenient restriction sites located near the promoter sequence to provide for the insertion of nucleic acid sequences encoding heterologous proteins. A selectable marker operative in the expression host may be present. Expression vectors may be used for the production of fusion proteins, where the exogenous fusion peptide provides additional functionality, i.e. increased protein synthesis, stability, reactivity with defined antisera, an enzyme marker, e.g. β-galactosidase, etc.

Expression cassettes may be prepared comprising a transcription initiation region, the gene or fragment thereof, and a transcriptional termination region. Of particular interest is the use of sequences that allow for the expression of functional epitopes or domains, usually at least about 8 amino acids in length, more usually at least about 15 amino acids in length, to about 25 amino acids, and up to the complete open reading frame of the gene. After introduction of the DNA, the cells containing the construct may be selected by means of a selectable marker, the cells expanded and then used for expression.

DGAT proteins and polypeptides may be expressed in prokaryotes or eukaryotes in accordance with conventional ways, depending upon the purpose for expression. For large scale production of the protein, a unicellular organism, such as *E. coli, B. subtilis, S. cerevisiae,* insect cells in combination with baculovirus vectors, or cells of a higher organism such as vertebrates, particularly mammals, *e.g.* COS 7 cells, may be used as the expression host cells. In some situations, it is desirable to express the *DGAT* gene in eukaryotic cells, where the DGAT protein will benefit from native folding and post-translational modifications. Small peptides can also be synthesized in the laboratory. Polypeptides that are subsets of the complete DGAT sequence may be used to identify and investigate parts of the protein important for function.

Once the source of the protein is identified and/or prepared, e.g. a transfected host expressing the protein is prepared, the protein is then purified to produce the desired DGAT comprising composition. Any convenient protein purification procedures may be employed, where suitable protein purification methodologies are described in Guide to Protein Purification, (Deuthser ed.) (Academic Press, 1990). For example, a lysate may prepared from the original source, e.g. naturally occurring cells or tissues that express DGAT or the expression host expressing DGAT, and purified using HPLC, exclusion chromatography, gel electrophoresis, affinity chromatography, and the like.

### METHODS AND COMPOSITIONS HAVING RESEARCH APPLICATION

Also disclosed are methods and compositions having research applications, such as in the study of the acylglycerol metabolism, in the identification of key components of the triglyceride synthesis pathway, in the identification of triglyceride synthesis modulatory agents, e.g. DGAT inhibitors or enhancers, and the like.

The subject nucleic acid compositions find use in a variety of research applications. Research applications of interest include: the identification of DGAT homologs; as a source of novel promoter elements; the identification of DGAT expression regulatory factors; as probes and primers in hybridization applications, e.g. PCR; the identification of expression patterns in biological specimens; the preparation of cell or animal models for DGAT function; the preparation of *in vitro* models for DGAT function; etc.

Homologs of *DGAT* are identified by any of a number of methods. A fragment of the provided cDNA may be used as a hybridization probe against a cDNA library from the target organism of interest, where low stringency conditions are used. The probe may be a large fragment, or one or more short degenerate primers. Nucleic acids having sequence similarity are detected by hybridization under low stringency conditions, for example, at 50°C and 6×SSC (0.9 M sodium chloride/0.09 M sodium citrate) and remain bound when subjected to washing at 55°C in 1×SSC (0.15 M sodium chloride/0.015 M sodium citrate). Sequence identity may be determined by hybridization under stringent conditions, for example, at 50°C or higher and 0.1×SSC (15 mM sodium chloride/01.5 mM sodium citrate). Nucleic acids having a region of substantial identity to the provided DGAT sequences, e.g. allelic variants, genetically altered versions of the gene, etc., bind to the provided DGAT sequences under stringent hybridization conditions. By using probes, particularly labeled probes of DNA sequences, one can isolate homologous or related genes. One can also use sequence information derived from the polynucleotide compositions of the subject invention to prepare electronic "probes" for use in searching of computer based. sequence date, e.g. BLAST searches EST databases.

The sequence of the 5' flanking region of the subject nucleic acid compositions may be utilized as a source for promoter elements, including enhancer binding sites, that provide for developmental regulation in tissues where DGAT is expressed. The tissue specific expression is useful for determining the pattern of expression, and for providing promoters that mimic the native pattern of expression. Naturally occurring polymorphisms in the promoter region are useful for determining natural variations in expression, particularly those that may be associated with disease.

Alternatively, mutations may be introduced into the promoter region to determine the effect of altering expression in experimentally defined systems. Methods for the identification of specific DNA motifs involved in the binding of transcriptional factors are known in the art, *e.g*. sequence similarity to known binding motifs, gel retardation studies, *etc.* For examples, see Blackwell et al. (1995), Mol. Med. 1:194-205; Mortlock et al. (1996), Genome Res. 6:327-33; and Joulin and Richard-Foy (1995), Eur. J. Biochem. 232:620-626.

The regulatory sequences may be used to identify *cis* acting sequences required for transcriptional or translational regulation of DGAT gene expression, especially in different tissues or stages of development, and to identify cis acting sequences and *trans*-acting factors that regulate or mediate DGAT gene expression. Such transcription or translational control regions may be operably linked to a DGAT gene in order to promote expression of wild type or altered DGAT or other proteins of interest in cultured cells, or in non-human embryonic, fetal or adult tissues, and for gene therapy.

Small DNA fragments are useful as primers for PCR, hybridization screening probes, *etc.* Larger DNA fragments, *i.e.* greater than 100 nt are useful for production of the encoded polypeptide, as described in the previous section. For use in amplification reactions, such as PCR, a pair of primers will be used. The exact composition of the primer sequences is not critical to the invention, but for most applications the primers will hybridize to the subject sequence under stringent conditions, as known in the art. It is preferable to choose a pair of primers that will generate an amplification product of at least about 50 nt, preferably at least about 100 nt. Algorithms for the selection of primer sequences are generally known, and are available in commercial software packages. Amplification primers hybridize to complementary strands of DNA, and will prime towards each other.

The DNA may also be used to identify expression of the gene in a biological specimen. The manner in which one probes cells for the presence of particular nucleotide sequences, as genomic DNA or RNA, is well established in the literature. Briefly, DNA or mRNA is isolated from a cell sample. The mRNA may be amplified by RT-PCR, using reverse transcriptase to form a complementary DNA strand, followed by polymerase chain reaction amplification using primers specific for the subject DNA sequences. Alternatively, the mRNA sample is separated by gel electrophoresis, transferred to a suitable support, *e.g.* nitrocellulose, nylon, *etc.,* and then probed with a fragment of the subject DNA as a probe. Other techniques, such as oligonucleotide ligation assays, *in situ* hybridizations, and hybridization to DNA probes arrayed on a solid chip may also find use. Detection of mRNA hybridizing to the subject sequence is indicative of DGAT gene expression in the sample.

The sequence of a DGAT gene, including flanking promoter regions and coding regions, may be mutated in various ways known in the art to generate targeted changes in promoter strength, sequence of the encoded protein, *etc.* The DNA sequence or protein product of such a mutation will usually be substantially similar to the sequences provided herein, *i.e.* will differ by at least one nucleotide or amino acid, respectively, and may differ by at least two but not more than about ten nucleotides or amino acids. The sequence changes may be substitutions, insertions, deletions, or a combination thereof. Deletions may further include larger changes, such as deletions of a domain or exon. Other modifications of interest include epitope tagging, *e.g.* with the FLAG system, HA, *etc.* For studies of subcellular localization, fusion proteins with green fluorescent proteins (GFP) may be used.

Techniques for *in vitro* mutagenesis of cloned genes are known. Examples of protocols for site specific mutagenesis may be found in Gustin et al. (1993), Biotechniques 14:22; Barany (1985), Gene 37:111-23; Colicelli et al. (1985), Mol. Gen. Genet. 199:537-9; and Prentki et al. (1984), Gene 29:303-13. Methods for site specific mutagenesis can be found in Sambrook et al., Molecular Cloning: A Laboratory Manual, CSH Press 1989, pp. 15.3-15.108; Weiner et al. (1993), Gene 126:35-41; Sayers et al. (1992), Biotechniques 13:592-6; Jones and Winistorfer (1992), Biotechniques 12:528-30; Barton et al. (1990), Nucleic Acids Res 18:7349-55; Marotti and Tomich (1989), Gene Anal. Tech. 6:67-70; and Zhu (1989), Anal Biochem 177:120-4. Such mutated genes may be used to study structure-function relationships of DGAT, or to alter properties of the protein that affect its function or regulation.

The subject nucleic acids can be used to generate transgenic hosts, e.g non-human animals, such as mice, cows, rats, pigs etc., plants, fungi, or site specific gene modifications in cell lines. Examples of transgenic hosts include host in which the naturally expressed DGAT gene has been disrupted, e.g. DGAT knock-outs, as well as hosts in which DGAT expression has been amplified, e.g. through introduction of additional DGAT copies, through introduction of strong promoter upstream of the DGAT gene, and the like. Using the nucleic acid compositions of the subject invention, standard protocols known to those of skill in the art may used to produce such transgenic hosts that have been genetically manipulated with respect to the DGAT gene, i.e. DGAT transgenic hosts.

Transgenic animals may be made through homologous recombination, where the normal DGAT locus is altered, e.g. as in DGAT knockouts. Alternatively, a nucleic acid construct is randomly integrated into the genome. Vectors for stable integration include plasmids, retroviruses and other animal viruses, YACs, and the like. DNA constructs for homologous recombination will comprise at least a portion of the DGAT gene native to the species of the host animal, wherein the gene has the desired genetic modification(s), and includes regions of homology to the target locus. DNA constructs for random integration need not include regions of homology to mediate recombination. Conveniently, markers for positive and negative selection are included. Methods for generating cells having targeted gene modifications through homologous recombination are known in the art. For various techniques for transfecting mammalian cells, see Keown et al. (1990), Meth. Enzymol. 185:527-537.

For non-human embryonic stem (ES) cells, an ES cell line may be employed, or embryonic cells may be obtained freshly from a host, *e.g.* mouse, rat, guinea pig, cow, *etc.* Such cells are grown on an appropriate fibroblast-feeder layer or grown in the presence of leukemia inhibiting factor (LIF). When non-human ES or non-human embryonic cells have been transformed, they may be used to produce transgenic animals. After transformation, the cells are plated onto a feeder layer in an appropriate medium. Cells containing the construct may be detected by employing a selective medium. After sufficient time for colonies to grow, they are picked and analyzed for the occurrence of homologous recombination or integration of the construct. Those colonies that are positive may then be used for embryo manipulation and blastocyst injection. Blastocysts are obtained from 4 to 6 week old superovulated females. The ES cells are trypsinized, and the modified cells are injected into the blastocoel of the blastocyst. After injection, the blastocysts are returned to each uterine horn of pseudopregnant females. Females are then allowed to go to term and the resulting offspring screened for the construct. By providing for a different phenotype of the blastocyst and the genetically modified cells, chimeric progeny can be readily detected.

The resultant chimeric animals are screened for the presence of the modified gene and males and females having the modification are mated to produce homozygous progeny. If the gene alterations cause lethality at some point in development, tissues or organs can be maintained as allogeneic or congenic grafts or transplants, or in *in vitro* culture. The transgenic animals may be any non-human mammal, such as laboratory animals, domestic animals, etc.

Transgenic plants may be produced in a similar manner. Methods of preparing transgenic plant cells and plants are described in U.S. Pat. Nos. 5,767,367; 5,750,870; 5,739,409; 5,689,049; 5,689,045; 5,674,731; 5,656,466; 5,633,155; 5,629,470; 5,595,896; 5,576,198; 5,538,879; 5,484,956; . Methods of producing transgenic plants are also reviewed in Plant Biochemistry and Molecular Biology (eds Lea & Leegood, John Wiley & Sons)(1993) pp 275-295. In brief, a suitable plant cell or tissue is harvested, depending on the nature of the plant species. As such, in certain instances, protoplasts will be isolated, where such protoplasts may be isolated from a variety of different plant tissues, e.g. leaf, hypoctyl, root, etc. For protoplast isolation, the harvested cells are incubated in the presence of cellulases in order to remove the cell wall, where the exact incubation conditions vary depending on the type of plant and/or tissue from which the cell is derived. The resultant protoplasts are then separated from the resultant cellular debris by sieving and centrifugation. Instead of using protoplasts, embryogenic explants comprising somatic cells may be used for preparation of the transgenic host. Following cell or tissue harvesting, exogenous DNA of interest is introduced into the plant cells, where a variety of different techniques are available for such introduction. With isolated protoplasts, the opportunity arise for introduction via DNA-mediated gene transfer protocols, including: incubation of the protoplasts with naked DNA, e.g. plasmids, comprising the exogenous coding sequence of interest in the presence of polyvalent cations, e.g. PEG or PLO; and electroporation of the protoplasts in the presence of naked DNA comprising the exogenous sequence of interest. Protoplasts that have successfully taken up the exogenous DNA are then selected, grown into a callus, and ultimately into a transgenic plant through contact with the appropriate amounts and ratios of stimulatory factors, e.g. auxins and cytokinins. With embryogenic explants, a convenient method of introducing the exogenous DNA in the target somatic cells is through the use of particle acceleration or "gene-gun" protocols. The resultant explants are then allowed to grow into chimera plants, cross-bred and transgenic progeny are obtained. Instead of the naked DNA approaches described above, another convenient method of producing transgenic plants is *Agrobacterium* mediated transformation. With *Agrobacterium* mediated transformation, co-integrative or binary vectors comprising the exogenous DNA are prepared and then introduced into an appropriate *Agrobacterium* strain, e.g. *A. tumefaciens.* The resultant bacteria are then incubated with prepared protoplasts or tissue explants, e.g. leaf disks, and a callus is produced. The callus is then grown under selective conditions, selected and subjected to growth media to induce root and shoot growth to ultimately produce a transgenic plant.

The modified cells, animals or plants are useful in the study of DGAT function and regulation. For example, a series of small deletions and/or substitutions may be made in the host's native DGAT gene to determine the role of different exons in various physiological processes. Specific constructs of interest include anti-sense DGAT, which will block DGAT expression, expression of dominant negative DGAT mutations, and over-expression of DGAT genes. Where a DGAT sequence is introduced, the introduced sequence may be either a complete or partial sequence of a DGAT gene native to the host, or may be a complete or partial DGAT sequence that is exogenous to the host animal, *e.g.*, a human DGAT sequence. A detectable marker, such as *lac* Z may be introduced into the DGAT locus, where upregulation of DGAT gene expression will result in an easily detected change in phenotype. One may also provide for expression of the DGAT gene or variants thereof in cells or tissues where it is not normally expressed, at levels not normally present in such cells or tissues, or at abnormal times of development. The transgenic hosts, e.g. animals, plants, etc., may be used in functional studies, drug screening, *etc., e.g.* to determine the effect of a candidate drug on DGAT activity, to identify drugs that reduce serum triglyceride levels, etc.

The subject polypeptide compositions can be used to produce *in vitro* models of triglyceride synthesis, where such models will consist of the subject DGAT proteins and other components of triglyceride synthesis, e.g. substrates, such as diacylglycerol or metabolic precersors thereof, fatty acyl CoAs and the like, other components of the triacylglycerol synthetase complex, e.g. acyl CoA ligase, acyl CoA acyltransferase, monoacyl glycerol acyltransferase, etc.

Also provided by the subject invention are screening assays designed to find modulatory agents of DGAT activity, e.g. inhibitors or enhancers of DGAT activity, as well as the agents identified thereby, where such agents may find use in a variety of applications, including as therapeutic agents, as agricultural chemicals, etc. The screening methods will typically be assays which provide for qualitative/quantitative measurements of DGAT activity in the presence of a particular candidate therapeutic agent. For example, the assay could be an assay which measures the acylation activity of DGAT in the presence and absence of a candidate inhibitor agent. The screening method may be an *in vitro* or *in vivo* format, where both formats are readily developed by those of skill in the art. Depending on the particular method, one or more of, usually one of, the components of the screening assay may be labeled, where by labeled is meant that the components comprise a detectable moiety, *e.g.* a fluorescent or radioactive tag, or a member of a signal producing system, *e.g.* biotin for binding to an enzyme-streptavidin conjugate in which the enzyme is capable of converting a substrate to a chromogenic product. Where in vitro assays are employed, the various components of the in vitro assay, e.g. the substrate, the donor, the DGAT protein and the candidate inhibitor, etc. are combined in a assay mixture under conditions sufficient for DGAT activity to occur, as described in the experimental section, *infra.*

A variety of other reagents may be included in the screening assay and reaction mixture. These include reagents like salts, neutral proteins, e.g. albumin, detergents, etc that are used to facilitate optimal protein-protein binding and/or reduce nonspecific or background interactions. Reagents that improve the efficiency of the assay, such as protease inhibitors, nuclease inhibitors, anti-microbial agents, *etc.* may be used.

A variety of different candidate agents may be screened by the above methods. Candidate agents encompass numerous chemical classes, though typically they are organic molecules, preferably small organic compounds having a molecular weight of more than 50 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof.

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

Using the above screening methods, a variety of different therapeutic agents may be identified. Such agents may target the enzyme itself, or an expression regulator factor thereof. Such agents may inhibitors or promoters of DGAT activity, where inhibitors are those agents that result in at least a reduction of DGAT activity as compared to a control and enhancers result in at least an increase in DGAT activity as compared to a control. Such agents may be find use in a variety of therapeutic applications, as described in greater detail below.

### METHODS AND COMPOSITIONS HAVING MEDICAL APPLICATIONS

The methods and compositions disclosed also have broad ranging applications in a variety of medical applications, including diagnostic screening, therapeutic treatments of pathological conditions, in the regulation of DGAT activity in desirable ways, and the like.

Disclosed are methods of screening individuals for a predisposition to a disease state or the presence of disease state, where such screening may focus on the presence of one or more markers, such as a mutated DGAT gene or expression regulatory element thereof, observed levels of DGAT; the expression level of the DGAT gene in a biological sample of interest; and the like.

Samples, as used herein, include biological fluids such as blood, cerebrospinal fluid, tears, saliva, lymph, semen, dialysis fluid and the like; organ or tissue culture derived fluids; and fluids extracted from physiological tissues. Also included in the term are derivatives and fractions of such fluids. The cells may be dissociated, in the case of solid tissues, or tissue sections may be analyzed. Alternatively a lysate of the cells may be prepared.

A number of methods are available for determining the expression level of a gene or protein in a particular sample. Diagnosis may be performed by a number of methods to determine the absence or presence or altered amounts of normal or abnormal DGAT in a patient sample. For example, detection may utilize staining of cells or histological sections with labeled antibodies, performed in accordance with conventional methods. Cells are permeabilized to stain cytoplasmic molecules. The antibodies of interest are added to the cell sample, and incubated for a period of time sufficient to allow binding to the epitope, usually at least about 10 minutes. The antibody may be labeled with radioisotopes, enzymes, fluorescers, chemiluminescers, or other labels for direct detection. Alternatively, a second stage antibody or reagent is used to amplify the signal. Such reagents are well known in the art. For example, the primary antibody may be conjugated to biotin, with horseradish peroxidase-conjugated avidin added as a second stage reagent. Alternatively, the secondary antibody conjugated to a flourescent compound, *e.g*. fluorescein, rhodamine, Texas red, *etc.* Final detection uses a substrate that undergoes a color change in the presence of the peroxidase. The absence or presence of antibody binding may be determined by various methods, including flow cytometry of dissociated cells, microscopy, radiography, scintillation counting, *etc*.

Alternatively, one may focus on the expression of *DGAT.* Biochemical studies may be performed to determine whether a sequence polymorphism in a *DGAT* coding region or control regions is associated with disease. Disease associated polymorphisms may include deletion or truncation of the gene, mutations that alter expression level, that affect the activity of the protein, *etc*.

Changes in the promoter or enhancer sequence that may affect expression levels of DGAT can be compared to expression levels of the normal allele by various methods known in the art. Methods for determining promoter or enhancer strength include quantitation of the expressed natural protein; insertion of the variant control element into a vector with a reporter gene such as β-galactosidase, luciferase, chloramphenicol acetyltransferase, *etc.* that provides for convenient quantitation; and the like.

A number of methods are available for analyzing nucleic acids for the presence of a specific sequence, *e.g.* a disease associated polymorphism. Where large amounts of DNA are available, genomic DNA is used directly. Alternatively, the region of interest is cloned into a suitable vector and grown in sufficient quantity for analysis. Cells that express DGAT may be used as a source of mRNA, which may be assayed directly or reverse transcribed into cDNA for analysis. The nucleic acid may be amplified by conventional techniques, such as the polymerase chain reaction (PCR), to provide sufficient amounts for analysis. The use of the polymerase chain reaction is described in Saiki, et al. (1985), Science 239:487, and a review of techniques may be found in Sambrook, et al. Molecular Cloning: A Laboratory Manual, CSH Press 1989, pp.14.2-14.33. Alternatively, various methods are known in the art that utilize oligonucleotide ligation as a means of detecting polymorphisms, for examples see Riley et al. (1990), Nucl. Acids Res. 18:2887-2890; and Delahunty et al. (1996), Am. J. Hum. Genet. 58:1239-1246.

A detectable label may be included in an amplification reaction. Suitable labels include fluorochromes, *e.g.* fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, *e.g.* ³²P, ³²S, ³H; *etc.* The label may be a two stage system, where the amplified DNA is conjugated to biotin, haptens, *etc.* having a high affinity binding partner, *e.g.* avidin, specific antibodies, *etc.,* where the binding partner is conjugated to a detectable label. The label may be conjugated to one or both of the primers. Alternatively, the pool of nucleotides used in the amplification is labeled, so as to incorporate the label into the amplification product.

The sample nucleic acid, *e.g*. amplified or cloned fragment, is analyzed by one of a number of methods known in the art. The nucleic acid may be sequenced by dideoxy or other methods, and the sequence of bases compared to a wild-type DGAT sequence. Hybridization with the variant sequence may also be used to determine its presence, by Southern blots, dot blots, *etc.* The hybridization pattern of a control and variant sequence to an array of oligonucleotide probes immobilized on a solid support, as described in US 5,445,934, or in WO 95/35505, may also be used as a means of detecting the presence of variant sequences. Single strand conformational polymorphism (SSCP) analysis, denaturing gradient gel electrophoresis (DGGE), and heteroduplex analysis in gel matrices are used to detect conformational changes created by DNA sequence variation as alterations in electrophoretic mobility. Alternatively, where a polymorphism creates or destroys a recognition site for a restriction endonuclease, the sample is digested with that endonuclease, and the products size fractionated to determine whether the fragment was digested. Fractionation is performed by gel or capillary electrophoresis, particularly acrylamide or agarose gels.

Screening for mutations in DGAT may be based on the functional or antigenic characteristics of the protein. Protein truncation assays are useful in detecting deletions that may affect the biological activity of the protein. Various immunoassays designed to detect polymorphisms in DGAT proteins may be used in screening. Where many diverse genetic mutations lead to a particular disease phenotype, functional protein assays have proven to be effective screening tools. The activity of the encoded DGAT protein may be determined by comparison with the wild-type protein.

Diagnostic methods in which the level of DGAT expression is of interest will typically involve comparison of the DGAT nucleic acid abundance of a sample of interest with that of a control value to determine any relative differences, where the difference may be measured qualitatively and/or quantitatively, which differences are then related to the presence or absence of an abnormal DGAT expression pattern. A variety of different methods for determining the nucleic acid abundance in a sample are known to those of skill in the art, where particular methods of interest include those described in: Pietu et al., Genome Res. (June 1996) 6: 492-503; Zhao et al., Gene (April 24, 1995) 156: 207-213; Soares , Curr. Opin. Biotechnol. (October 1997) 8: 542-546; Raval, J. Pharmacol Toxicol Methods (November 1994) 32: 125-127; Chalifour et al., Anal. Biochem (February 1, 1994) 216: 299-304; Stolz & Tuan, Mol. Biotechnol. (December 19960 6: 225-230; Hong et al., Bioscience Reports (1982) 2: 907; and McGraw, Anal. Biochem. (1984) 143: 298. Also of interest are the methods disclosed in WO 97/27317 .

The subject diagnostic or screening methods may be used to identify the presence of, or predisposition to, disease conditions associated with acylglycerol metabolism, particularly those associated with DGAT and/or DGAT-2 activity. Such disease conditions include: hyperlipidemia (including excess serum triglyceride levels), cardiovascular disease, obesity, diabetes, cancer, neurological disorders, immunological disorders, and the like.

Also disclosed are methods of regulating, including enhancing and inhibiting, DGAT activity in a host. A variety of situations arise where modulation of DGAT activity in a host is desired, where such conditions include disease conditions associated with DGAT activity and non-disease conditions in which a modulation of DGAT activity is desired for a variety of different reasons, e.g. cosmetic weight control.

For the modulation of DGAT activity in a host, an effective amount of active agent that modulates the activity, e.g. reduces the activity, of DGAT *in vivo*, is administered to the host. The active agent may be a variety of different compounds, including: the polynucleotide compositions of the subject invention, the polypeptide compositions of the subject invention, a naturally occurring or synthetic small molecule compound, an antibody, fragment or derivative thereof, an antisense composition, and the like.

The nucleic acid compositions disclosed find use as therapeutic agents in situations where one wishes to enhance DGAT activity in a host, e.g. in a mammalian host in which DGAT activty is low resulting in a disease condition, etc. The DGAT genes, gene fragments, or the encoded DGAT protein or protein fragments are useful in gene therapy to treat disorders associated with DGAT defects. Expression vectors may be used to introduce the DGAT gene into a cell. Such vectors generally have convenient restriction sites located near the promoter sequence to provide for the insertion of nucleic acid sequences. Transcription cassettes may be prepared comprising a transcription initiation region, the target gene or fragment thereof, and a transcriptional termination region. The transcription cassettes may be introduced into a variety of vectors, *e.g.* plasmid; retrovirus, *e.g.* lentivirus; adenovirus; and the like, where the vectors are able to transiently or stably be maintained in the cells, usually for a period of at least about one day, more usually for a period of at least about several days to several weeks.

Naturally occurring or synthetic small molecule compounds of interest include numerous chemical classes, though typically they are organic molecules, preferably small organic compounds having a molecular weight of more than 50 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof. Of particular interest are those agents identified by the screening assays of the subject invention, as described above.

Also of interest as active agents are antibodies that modulate, e.g. reduce, if not inhibit, DGAT activity in the host. Suitable antibodies are obtained by immunizing a host animal with peptides comprising all or a portion of a DGAT protein, such as the DGAT polypeptide compositions of the subject invention. Suitable host animals include mouse, rat sheep, goat, hamster, rabbit, *etc.* The origin of the protein immunogen may be mouse, human, rat, monkey *etc.* The host animal will generally be a different species than the immunogen, *e.g.* human DGAT used to immunize mice, *etc.*

The immunogen may comprise the complete protein, or fragments and derivatives thereof. Preferred immunogens comprise all or a part of DGAT, where these residues contain the post-translation modifications, such as glycosylation, found on the native DGAT. Immunogens comprising the extracellular domain are produced in a variety of ways known in the art, *e.g.* expression of cloned genes using conventional recombinant methods, isolation from HEC, *etc.*

For preparation of polyclonal antibodies, the first step is immunization of the host animal with DGAT, where the DGAT will preferably be in substantially pure form, comprising less than about 1% contaminant. The immunogen may comprise complete DGAT, fragments or derivatives thereof. To increase the immune response of the host animal, the DGAT may be combined with an adjuvant, where suitable adjuvants include alum, dextran, sulfate, large polymeric anions, oil & water emulsions, e.g. Freund's adjuvant, Freund's complete adjuvant, and the like. The DGAT may also be conjugated to synthetic carrier proteins or synthetic antigens. A variety of hosts may be immunized to produce the polyclonal antibodies. Such hosts include rabbits, guinea pigs, rodents, e.g. mice, rats, sheep, goats, and the like. The DGAT is administered to the host, usually intradermally, with an initial dosage followed by one or more, usually at least two, additional booster dosages. Following immunization, the blood from the host will be collected, followed by separation of the serum from the blood cells. The Ig present in the resultant antiserum may be further fractionated using known methods, such as ammonium salt fractionation, DEAE chromatography, and the like.

Monoclonal antibodies are produced by conventional techniques. Generally, the spleen and/or lymph nodes of an immunized host animal provide a source of plasma cells. The plasma cells are immortalized by fusion with myeloma cells to produce hybridoma cells. Culture supernatant from individual hybridomas is screened using standard techniques to identify those producing antibodies with the desired specificity. Suitable animals for production of monoclonal antibodies to the human protein include mouse, rat, hamster, *etc.* To raise antibodies against the mouse protein, the animal will generally be a hamster, guinea pig, rabbit, etc. The antibody may be purified from the hybridoma cell supernatants or ascites fluid by conventional techniques, *e.g.* affinity chromatography using DGAT bound to an insoluble support, protein A sepharose, *etc.*

The antibody may be produced as a single chain, instead of the normal multimeric structure. Single chain antibodies are described in Jost et al. (1994) J.B.C. 269:26267-73, and others. DNA sequences encoding the variable region of the heavy chain and the variable region of the light chain are ligated to a spacer encoding at least about 4 amino acids of small neutral amino acids, including glycine and/or serine. The protein encoded by this fusion allows assembly of a functional variable region that retains the specificity and affinity of the original antibody.

For *in vivo* use, particularly for injection into humans, it is desirable to decrease the antigenicity of the antibody. An immune response of a recipient against the blocking agent will potentially decrease the period of time that the therapy is effective. Methods of humanizing antibodies are known in the art. The humanized antibody may be the product of an animal having transgenic human immunoglobulin constant region genes (see for example International Patent Applications WO 90/10077 and WO 90/04036). Alternatively, the antibody of interest may be engineered by recombinant DNA techniques to substitute the CH1, CH2, CH3, hinge domains, and/or the framework domain with the corresponding human sequence (see WO 92/02190).

The use of Ig cDNA for construction of chimeric immunoglobulin genes is known in the art (Liu et al. (1987) P.N.A.S. 84:3439 and (1987) J. Immunol. 139:3521). mRNA is isolated from a hybridoma or other cell producing the antibody and used to produce cDNA. The cDNA of interest may be amplified by the polymerase chain reaction using specific primers (U.S. Patent nos. 4,683,195 and 4,683,202). Alternatively, a library is made and screened to isolate the sequence of interest. The DNA sequence encoding the variable region of the antibody is then fused to human constant region sequences. The sequences of human constant regions genes may be found in Kabat et al. (1991) Sequences of Proteins of Immunological Interest, N.I.H. publication no. 91-3242. Human C region genes are readily available from known clones. The choice of isotype will be guided by the desired effector functions, such as complement fixation, or activity in antibody-dependent cellular cytotoxicity. Preferred isotypes are IgG1, IgG3 and IgG4. Either of the human light chain constant regions, kappa or lambda, may be used. The chimeric, humanized antibody is then expressed by conventional methods.

Antibody fragments, such as Fv, F(ab')₂ and Fab may be prepared by cleavage of the intact protein, *e.g.* by protease or chemical cleavage. Alternatively, a truncated gene is designed. For example, a chimeric gene encoding a portion of the F(ab')₂ fragment would include DNA sequences encoding the CH1 domain and hinge region of the H chain, followed by a translational stop codon to yield the truncated molecule.

Consensus sequences of H and L J regions may be used to design oligonucleotides for use as primers to introduce useful restriction sites into the J region for subsequent linkage of V region segments to human C region segments. C region cDNA can be modified by site directed mutagenesis to place a restriction site at the analogous position in the human sequence.

Expression vectors include plasmids, retroviruses, YACs, EBV derived episomes, and the like. A convenient vector is one that encodes a functionally complete human CH or CL immunoglobulin sequence, with appropriate restriction sites engineered so that any VH or VL sequence can be easily inserted and expressed. In such vectors, splicing usually occurs between the splice donor site in the inserted J region and the splice acceptor site preceding the human C region, and also at the splice regions that occur within the human CH exons. Polyadenylation and transcription termination occur at native chromosomal sites downstream of the coding regions. The resulting chimeric antibody may be joined to any strong promoter, including retroviral LTRs, *e.g.* SV-40 early promoter, (Okayama et al. (1983) Mol. Cell. Bio. 3:280), Rous sarcoma virus LTR (Gorman et al. (1982) P.N.A.S. 79:6777), and moloney murine leukemia virus LTR (Grosschedl et al. (1985) Cell 41:885); native Ig promoters, *etc.*

Also the active agent may be an agent that modulates, and generally decreases or down regulates, the expression of *DGAT* in the host. Antisense molecules can be used to down-regulate expression of *DGAT* in cells. The anti-sense reagent may be antisense oligonucleotides (ODN), particularly synthetic ODN having chemical modifications from native nucleic acids, or nucleic acid constructs that express such anti-sense molecules as RNA. The antisense sequence is complementary to the mRNA of the targeted gene, and inhibits expression of the targeted gene products. Antisense molecules inhibit gene expression through various mechanisms, *e.g*. by reducing the amount of mRNA available for translation, through activation of RNAse H, or steric hindrance. One or a combination of antisense molecules may be administered, where a combination may comprise multiple different sequences.

Antisense molecules may be produced by expression of all or a part of the target gene sequence in an appropriate vector, where the transcriptional initiation is oriented such that an antisense strand is produced as an RNA molecule. Alternatively, the antisense molecule is a synthetic oligonucleotide. Antisense oligonucleotides will generally be at least about 7, usually at least about 12, more usually at least about 20 nucleotides in length, and not more than about 500, usually not more than about 50, more usually not more than about 35 nucleotides in length, where the length is governed by efficiency of inhibition, specificity, including absence of cross-reactivity, and the like. It has been found that short oligonucleotides, of from 7 to 8 bases in length, can be strong and selective inhibitors of gene expression (see Wagner et al. (1996), Nature Biotechnol. 14:840-844)*.*

A specific region or regions of the endogenous sense strand mRNA sequence is chosen to be complemented by the antisense sequence. Selection of a specific sequence for the oligonucleotide may use an empirical method, where several candidate sequences are assayed for inhibition of expression of the target gene in an *in vitro* or animal model. A combination of sequences may also be used, where several regions of the mRNA sequence are selected for antisense complementation.

Antisense oligonucleotides may be chemically synthesized by methods known in the art (see Wagner *et al.* (1993), *supra,* and Milligan *et al., supra.*) Preferred oligonucleotides are chemically modified from the native phosphodiester structure, in order to increase their intracellular stability and binding affinity. A number of such modifications have been described in the literature, which alter the chemistry of the backbone, sugars or heterocyclic bases.

Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include 3'-O'-5'-S-phosphorothioate, 3'-S-5'-O-phosphorothioate, 3'-CH2-5'-O-phosphonate and 3'-NH-5'-O-phosphoroamidate. Peptide nucleic acids replace the entire ribose phosphodiester backbone with a peptide linkage. Sugar modifications are also used to enhance stability and affinity. The α-anomer of deoxyribose may be used, where the base is inverted with respect to the natural β-anomer. The 2'-OH of the ribose sugar may be altered to form 2'-O-methyl or 2'-O-allyl sugars, which provides resistance to degradation without comprising affinity. Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine. 5- propynyl-2'-deoxyuridine and 5-propynyl-2'-deoxycytidine have been shown to increase affinity and biological activity when substituted for deoxythymidine and deoxycytidine, respectively.

As an alternative to anti-sense inhibitors, catalytic nucleic acid compounds, *e.g.* ribozymes, anti-sense conjugates, *etc.* may be used to inhibit gene expression. Ribozymes may be synthesized *in vitro* and administered to the patient, or may be encoded on an expression vector, from which the ribozyme is synthesized in the targeted cell (for example, see International patent application WO 9523225, and Beigelman et al. (1995), Nucl. Acids Res. 23:4434-42). Examples of oligonucleotides with catalytic activity are described in WO 9506764. Conjugates of anti-sense ODN with a metal complex, *e.g.* terpyridylCu(II), capable of mediating mRNA hydrolysis are described in Bashkin et al. (1995), Appl. Biochem: Biotechnol. 54:43-56.

As mentioned above, an effective amount of the active agent is administered to the host, where "effective amount" means a dosage sufficient to produce a desired result, where the desired result in the desired modulation, e.g. enhancement, reduction, of DGAT activity.

In the subject methods, the active agent(s) may be administered to the host using any convenient means capable of resulting in the desired effect. Thus, the agent can be incorporated into a variety of formulations for therapeutic administration. More particularly, the agents of the present invention can be formulated into pharmaceutical compositions by combination with appropriate, pharmaceutically acceptable carriers or diluents, and may be formulated into preparations in solid, semi-solid, liquid or gaseous forms, such as tablets, capsules, powders, granules, ointments, solutions, suppositories, injections, inhalants and aerosols.

As such, administration of the agents can be achieved in various ways, including oral, buccal, rectal, parenteral, intraperitoneal, intradermal, transdermal, intracheal,etc., administration.

In pharmaceutical dosage forms, the agents may be administered in the form of their pharmaceutically acceptable salts, or they may also be used alone or in appropriate association, as well as in combination, with other pharmaceutically active compounds. The following methods and excipients are merely exemplary and are in no way limiting.

For oral preparations, the agents can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The agents can be formulated into preparations for injection by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

The agents can be utilized in aerosol formulations to be administered via inhalation. The compounds of the present invention can be formulated into pressurized acceptable propellants such as dichlorodifluoromethane, propane, nitrogen and the like.

Furthermore, the agents can be made into suppositories by mixing with a variety of bases such as emulsifying bases or water-soluble bases. The compounds of the present invention can be administered rectally via a suppository. The suppository can include vehicles such as cocoa butter, carbowaxes and polyethylene glycols, which melt at body temperature, yet are solidified at room temperature.

Unit dosage forms for oral or rectal administration such as syrups, elixirs, and suspensions may be provided wherein each dosage unit, for example, teaspoonful, tablespoonful, tablet or suppository, contains a predetermined amount of the composition containing one or more inhibitors. Similarly, unit dosage forms for injection or intravenous administration may comprise the inhibitor(s) in a composition as a solution in sterile water, normal saline or another pharmaceutically acceptable carrier.

The term "unit dosage form," as used herein, refers to physically discrete units suitable as unitary dosages for human and animal subjects, each unit containing a predetermined quantity of compounds of the present invention calculated in an amount sufficient to produce the desired effect in association with a pharmaceutically acceptable diluent, carrier or vehicle. The specifications for the novel unit dosage forms of the present invention depend on the particular compound employed and the effect to be achieved, and the pharmacodynamics associated with each compound in the host.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

Where the agent is a polypeptide, polynucleotide, analog or mimetic thereof, e.g. antisense composition, it may be introduced into tissues or host cells by any number of routes, including viral infection, microinjection, or fusion of vesicles. Jet injection may also be used for intramuscular administration, as described by Furth et al. (1992), Anal Biochem 205:365-368. The DNA may be coated onto gold microparticles, and delivered intradermally by a particle bombardment device, or "gene gun" as described in the literature (see, for example, Tang et al. (1992), Nature 356:152-154), where gold microprojectiles are coated with the *DGAT* DNA, then bombarded into skin cells.

Those of skill will readily appreciate that dose levels can vary as a function of the specific compound, the severity of the symptoms and the susceptibility of the subject to side effects. Preferred dosages for a given compound are readily determinable by those of skill in the art by a variety of means.

The subject methods find use in the treatment of a variety of different disease conditions involving acylglycerol metabolism, and particularly DGAT activity, including both insufficient or hypo-DGAT activity and hyper-DGAT activity. Representative diseases that may be treated according to the subject methods include: hyperlipidemia (including excess serum triglyceride levels), cardiovascular disease, obesity, diabetes, cancer, neurological disorders, immunological disorders, skin disorders associated with sebaceous gland activity, e.g. acne, and the like.

By treatment is meant at least an amelioration of the symptoms associated with the pathological condition afflicting the host, where amelioration is used in a broad sense to refer to at least a reduction in the magnitude of a parameter, *e.g*. symptom, associated with the pathological condition being treated, such as serum triglyceride level, weight, total body fat content, etc. As such, treatment also includes situations where the pathological condition, or at least symptoms associated therewith, are completely inhibited, *e.g*. prevented from happening, or stopped, *e.g*. terminated, such that the host no longer suffers from the pathological condition, or at least the symptoms that characterize the pathological condition. For example, where the disease condition is marked by the presence of elevated lipid levels, treatment includes at least a reduction in the observed lipid levels, including a restoration of normal lipid levels. As another example, where the disease is obesity, treatment results in at least a reduction in the overall weight and/or total body fat content of the host.

The subject methods also find use in the modulation of DGAT activity in hosts not suffering from a disease condition but in which the modulation of DGAT activity is nonetheless desired. For example, sperm production in males has been associated with DGAT activity. As such, in males where at least reduced sperm production is desired, the subject methods can be used to reduce DGAT activity in such males, e.g. by administering an agent that reduces DGAT activity in such males, where such agents are described above. In other words, the subject methods provide a means of male contraception. Alternatively, where increased sperm count in a given male is desired, e.g. in those conditions where the male has reduced fertility, the subject methods can be used to enhance DGAT activity in the male and thereby increase sperm count and fertility, e.g. by administering to the male host a DGAT enhancing agent, as described above.

A variety of hosts are treatable according to the subject methods. Generally such hosts are "mammals" or "mammalian," where these terms are used broadly to describe organisms which are within the class mammalia, including the orders carnivore (*e.g*., dogs and cats), rodentia (*e.g*., mice, guinea pigs, and rats), and primates (*e.g*., humans, chimpanzees, and monkeys). In many embodiments, the hosts will be humans.

Kits with unit doses of the active agent, usually in oral or injectable doses, are provided. In such kits, in addition to the containers containing the unit doses will be an informational package insert describing the use and attendant benefits of the drugs in treating pathological condition of interest. Preferred compounds and unit doses are those described herein above.

### METHODS AND COMPOSITIONS FOR PRODUCING TRIGLYCERIDES AND TRIGLYCERIDE COMPOSITIONS

Also disclosed are methods for preparing triglycerides and triglyceride comprising compositions, as well as the compositions produced by these methods. In preparing triglycerides with the subject invention, at least the direct substrates of the desired triacylglyercol, e.g. diacylglycerol and fatty acyl CoA, will be combined in the presence of the polypeptide under conditions sufficient for the acylation of the diacylglycerol to occur. The synthesis may occur in an *in vitro* system, e.g. in a vessel in which the substrates or precursors thereof and the DGAT enzyme, as well as any other requisite enzymes (e.g. as need to convert the substrate precursors to substrates), or an *in vivo* system, e.g. a host cell that naturally comprises the substrates and into which a DGAT gene has been inserted in a manner sufficient for expression of the DGAT gene, where the resultant triglyceride products may be separated from the host cell using standard separation techniques.

Of interest for use in producing triglyceride compositions are transgenic plants/fungi that have been genetically manipulated using the nucleic acid compositions disclosed to produce triglycerides and/or compositions thereof in one or more desirable ways. Transgenic plants/fungi are those plants/fungi that at least: (a) produce more triglyceride or triglyceride composition than wild type, e.g. produce more oil, such as by producing seeds having a higher oil content, as compared to wild-type; (b) produce triglyceride compositions, e.g. oils, that are enriched for triglycerides and/or enriched for one or more particular triglycerides as compared to wild type; and the like. Of interest are transgenic plants that produce commercially valuable triglyceride compositions or oils, such as canola, rapeseed, palm, corn, etc., containing various poly- and mono-unsaturated fatty acids, and the like. Of particular interest are transgenic plants, such as canola, rapeseed, palm, oil, etc., which have been genetically modified to produce seeds having higher oil content than the content found in the corresponding wild type, where the oil content of the seeds produced by such plants is at least 10% higher, usually at least 20 % higher, and in many embodiments at least 30 % higher than that found in the wild type, where in many embodiments seeds having oil contents that are 50% higher, or even greater, as compared to seeds produced by the corresponding wild-type plant, are produced. The seeds produced by such DGAT transgenic plants can be used as sources of oil or as sources of additional DGAT transgenic plants. Such transgenic plants and seeds therefore find use in methods of producing oils. In such methods, DGAT transgenic plants engineered to produce seeds having a higher oil content than the corresponding wild-type, e.g. seeds in which the DGAT gene is overexpressed, are grown, the seeds are harvested and then processed to recover the oil. The subject transgenic plants can also be used to produce novel oils characterized by the presence of triglycerides in different amounts and/or ratios than those observed in naturally occurring oils. The transgenic plants/fungi described above can be readily produced by those of skill in the art armed with the nucleic acid compositions of the subject invention. *See* the discussion on how to prepare transgenic plants, *supra.*

The triglyceride compositions described above find use in a variety of different applications. For example, such compositions or oils find use as food stuffs, being used as ingredients, spreads, cooking materials, etc. Alternatively, such oils find use as industrial feedstocks for use in the production of chemicals, lubricants, surfactants and the like.

Also of interest are transgenic non-human animals suitable for use as sources of food products and/or animal based industrial products. Such trans-genic non-human animals, e.g. transgenic mice, rats, livestock, such as cows, pigs, horses, birds, etc, may be produced using methods known in the art and reviewed *supra.* Such trans-genic non-human animals can be used for sources of a variety of different food and industrial products in which the triglyceride content is specifically tailored in a desirable manner. For example, such trans-genic animals that have been modified in a manner such that DGAT activity is reduced as compared to the wild type can be used as sources of food products that are low in triglyceride content, e.g. low fat or lean meat products, low fat milk, low fat eggs, and the like.

The following examples are offered primarily for purposes of illustration. It will be readily apparent to those skilled in the art that the formulations, dosages, methods of administration, and other parameters of this invention may be further modified or substituted in various ways.

### EXPERIMENTAL

### I. Identification and Characterization of DGAT

### A. Cloning of DGAT cDNA.

ESTs (accession numbers R07932 (human) and W10786 (mouse)) with sequence similarity to ACAT were identified from BLAST searches of the databases. The 5' end of the DGAT cDNA was obtained by using 5' RACE and a mouse spleen Marathon Ready^{™} cDNA library (Clontech, Palo Alto, CA). The sequences were deposited in GenBank (accession no. AF078752).

The translation of a full-length cDNA for the mouse EST (SEQ ID NO.: 03) predicts an open reading frame encoding a 498-amino acid protein that is ∼20% identical to mouse ACAT, with the most highly conserved regions in the C-terminus. The predicted protein sequence (SEQ ID NO:07) contains a potential N-linked glycosylation site and a putative tyrosine phosphorylation site. A serine residue found in ACAT that is necessary for enzyme activity (as reported in Cao et al., J. Biol. Chem. (1996) 271:14642-14648) appears to be conserved. The protein has multiple hydrophobic domains and 6-12 possible transmembrane domains. Analysis by a transmembrane region prediction program (http://ulrec3.unil.ch/software/TMPRED_form.html) favors nine transmembrane domains (amino acids 96-114, 140-157, 174-198, 200-218, 293-311, 337-360, 412-434, 436-456, and 461-484).
**B. Insect cell expression studies.** DGAT coding sequences with or without an N-terminal FLAG epitope (IBI, Kodak, New Haven, CT) (MGDYKDDDDG-, epitope underlined. (SEQ ID NO:08)) were subcloned into the baculovirus transfer vector pVL1392 (PharMingen, San Diego, CA). High titers of recombinant baculoviruses were obtained by cotransfection of ACAT or DGAT baculovirus transfer vectors with viral BaculoGold^{™} DNA (PharMingen), followed by plaque purification and virus amplification in Sf9 cells (cultured in Grace's medium (Life Technologies, Grand Island, NY) and 10% fetal bovine serum). H5 insect cells (cultured in serum-free Express-Five medium (Life Technologies)) were plated on day 0 (8.5 x 10⁶ cells/100-mm dish) and infected on day 1 with high titers of virus at a multiplicity of infection (MOI) that was empirically determined. On day 3, cells were collected by centrifugation and washed twice with phosphate-buffered saline (PBS). Cell pellets were homogenized by 10 passages through a 27-G needle in 0.1 M sucrose, 50 mM KCl, 40 mM KH₂PO₄, 30 mM EDTA (pH 7.2). Total membrane fractions (100,000 x g pellet) were resuspended in the homogenization buffer and frozen (-80°C). Immunoblots of membrane proteins (75 µg) were performed using the anti-FLAG M2 monoclonal antibody (IBI, Kodak).

For metabolic labeling experiments, H5 insect cells were plated on day 0 at 2.9 × 10⁶ cells per 60-mm dish and infected on day 1 with high titers of viruses. On day 3, cells were washed and incubated in methionine- and cysteine-free medium (SF900 II, Life Technologies) for 2 h, followed by incubation in the same medium containing 715 µCi of [³⁵S]methionine and [³⁵S]cysteine (Amersham Pro-Mix) for 1 h. Cells were washed twice with PBS, collected by low-speed centrifugation, and the cell pellet was resuspended in 0.5 ml of 50 mM Tris-HCl, 150 mM NaCl, 5 mM EDTA, 1 mM PMSF, 1% Triton X-100 (pH 7.4) and sonicated. Cellular protein samples (100 mg) were analyzed by SDS-PAGE and autoradiography.

For ACAT assays, cell-membrane proteins (100 µg) were assayed by using [1-¹⁴C]oleoyl CoA (51 mCi/mmol, Amersham, Arlington Heights, IL) and cholesterol/egg phosphatidyl choline (PC) liposomes (molar ratio = 0.7) as described in Smith et al., J. Lipids Res. (1995) 36:641-652. In some assays, other acyl acceptors were substituted for cholesterol in the liposomes at a 0.2 molar ratio (acceptor:egg PC) to test their ability to act as substrates. Incorporation of the [¹⁴C]oleoyl group into products was assessed by thin-layer chromatography, followed by autoradiography. DGAT assays were based on assays optimized for rat liver. See Andersson supra, Ozasa et al., J. Lipids Res. (1989) 30:1759-1762; and S.K. Erickson, K. Pella, S. Lear, manuscript in preparation. The incorporation of [¹⁴C]oleoyl CoA into triacylglycerol was measured under apparent V_{MAX} conditions by using exogenous diacylglycerol provided as diacylglycerol:egg PC liposomes (molar ratio ∼0.16). Cell-membrane proteins (20-25 µg) were assayed in 0.25 M sucrose, 1 mM EDTA, 150 mM MgCl₂, 100 mM Tris-HCl (pH 7.5) containing 250 µg of bovine serum albumin and 20 µg of diacylglycerol in liposomes (final volume = 0.2 ml) and 5 nmol [¹⁴C]oleoyl CoA (40,000 dpm/nmol). Reactions were carried out for 5 min, and the products analyzed as described in Erickson et al., J. Lipids Res. (1980) 21: 930-941. Similar assays were performed with 1-stearoyl-2-[1-¹⁴C]arachidonyl-*sn*-glycerol (53 mCi/mmol, Amersham) diluted to a final activity of 38,000 dpm/nmol with unlabeled 1,2-diacyl-sn-glycerol and unlabeled oleoyl CoA.

Relative triacylglycerol and DAG masses were determined by total lipid extraction of membranes or cells followed by thin-layer chromatography, iodine vapor visualization, photography of the plates, and densitometric analysis. Triolein standards were used to estimate mass of triacylglycerols, and DAG units were estimated relative to one another. Triacylglycerol values were normalized to 1 for wild-type virus-infected cell membranes to correct for interexperiment variability.

Cells infected with the virus containing DGAT cDNA expressed a ∼47-kDa protein at high levels in the membrane fraction, but lacked detectable cholesterol esterification activity as compared with ACAT virus-infected cells. Using a variety of other possible acyl acceptors as provided substrates (including 25-hydroxy-, 27-hydroxy-, 7α-hydroxy- or 7β-hydroxycholesterols, 7-ketocholesterol, vitamins D2 and D3, vitamin E, dehydroepiandrosterone, retinol, ethanol, sitosterol, lanosterol, and ergosterol), no acyltransferase activity was detected in H5 membranes expressing the protein, as assessed by autoradiography of thin-layer chromatography plates used to analyze reaction products. However, further analysis of these plates revealed that membranes from these cells had significantly increased triacylglycerol mass (as assessed by I₂ visualization) and incorporated significantly more [¹⁴C]oleoyl CoA into triacylglycerols than did membranes from wild-type virus-infected cells (197 *vs.* 55 pmol/mg prot/min). These data suggested that the identified cDNA might encode a DGAT.

Measurements of DGAT activity in membranes from H5 insect cells expressing the putative DGAT cDNA revealed that DGAT activity in these membranes was more than fivefold higher than that in membranes from wild-type virus-infected cells. The DGAT activity level increased proportionately with the amount of FLAG-tagged protein expressed in membranes isolated from cells harvested at different time points following infection. DGAT activity levels were similar regardless of whether [¹⁴C]diacylglycerol or [¹⁴C]oleoyl CoA was used as the labeled substrate. In the absence of added oleoyl CoA, [¹⁴C]diacylglycerol was not incorporated into triacylglycerols. Additionally, [³H]oleic acid was not incorporated into triacylglycerols in DGAT virus-infected membranes (7 ± 6 *vs.* 49 ± 47 pmol triacylglycerol/mg prot/min for wild-type, *n* = 3), establishing the requirement for a fatty acyl CoA. Triacylglycerol mass was more than 10-fold higher in membranes from DGAT virus-infected cells than in membranes from wild-type virus-infected cells (11 ± 7 *vs.* 1 ± 0.5 pg/µg membrane protein, *P* = 0.04, *n* = 5). No change in relative DAG mass was observed (0.33 ± 0.05 vs. 0.34 ± 0.12 units for DGAT and wild-type, respectively).
**C. mRNA expression.** Human Multiple Tissue northern blots (Clontech) were hybridized with a ³²P-labeled 1.1-kb human DGAT fragment from the human EST. For mouse tissues, total RNA was prepared with Trizol reagent (Life Technologies), and samples (10 mg) were analyzed by northern blotting using a ³²P-labeled, 1-kb mouse DGAT fragment from the mouse EST. Blots were stripped and sequentially reprobed for G3PDH and 28S RNA as described in Barbu & Dautry, Nucleic Acids Res. (1989)17:7115. Bands in autoradiograms from the 3T3-L1 experiments were quantified with a phosphoimager (Fuji Medical Systems, Stamford, CT).

mRNA expression was detected in every human and mouse tissue examined, as expected for DGAT's role in cellular glycerolipid metabolism. The highest expression levels were found in the small intestine, consistent with a proposed role for DGAT in intestinal fat absorption (see Brindley supra, and Mansbach, Biochim. Biophys. Acta (1973) 296:386-402. Additionally, mRNA was expressed in mouse adipose tissue, a tissue known to have high DGAT activity (see Coleman & Bell, supra). Interestingly, mRNA expression was not particularly high in the livers of humans or mice, despite the fact that liver tissue possesses DGAT activity (as reported in Polokoff & Bell, Biochim. Biophys. Acta (1980)35:535-545.
**D. NIH 3T3-L1 differentiation.** NIH 3T3-L1 fibroblasts were cultured in Dulbecco's Modified Eagle's medium supplemented with 10% fetal bovine serum, 100 units/ml penicillin, 100 µg/ml streptomycin, and 2 mM L-glutamine. The differentiation of 3T3-L1 cells into adipocytes was induced by incubating confluent monolayers of cells in serum-containing medium supplemented with 10⁻⁵ M dexamethasone, 0.5 mM isobutylmethylxanthine, and 10 µg/ml insulin as described Brasaemle et al., J. Lipid Res. (1997) 38:2249-2263.

It was found that mRNA expression increased markedly in parallel with DGAT activity in NIH 3T3-L1 cells during their differentiation into adipocytes, a process known to be associated with increases in DGAT activity (see Coleman et al., J. Biol. Chem. (1978) 253:7256-7261) and triacylglycerol mass accumulation (see Green & Kehinde, Cell (1975) 5:19-27.

### E. Intermediate Conclusions

The above results indicate that the cDNA encodes a DGAT protein. As a final piece of evidence confirming the identity of this cDNA, the mouse DGAT gene in embryonic stem cells was disrupted and germline transmission of this mutation was achieved. In preliminary experiments, the DGAT activity in membranes from embryonic fibroblasts homozygous for the knockout mutation (-/-) are markedly reduced compared with that in wild-type fibroblasts (25.5 ± 2.6 vs. 453.6 ± 4.5 pmol triacylglycerol/mg prot/min for -/- and +/+, respectively; *P* < 0.001). Taken together, the experimental data indicate that the identified cDNA encodes a DGAT catalytic unit.
**F. Gene mapping.** Primers derived from the human EST sequences were used to identify genomic clones in an arrayed BAC library according to manufacturer's protocol (Research Genetics, Huntsville, AL). The BAC clone was mapped to chromosome 8qter by fluorescent *in situ* hybridization as described in Stokke et al., Genomics (1995) 26:134-137. The clone (RMC08P049) may be requested from the website (http://rmc-www.lbl.gov). Linkage analysis for mouse gene mapping was performed with a panel of 67 progeny derived from a ((C57BL/6J x *Mus spretus*) F1 × C57BL/6J) interspecific backcross as described in Warden et al., Genomics (1993) 18:295-307. This backcross panel has been_typed for more than 400 loci distributed throughout the genome. See Welch et al., J. Lipid Res. (1996) 37:1406-1421. Briefly, parental strain DNAs were screened for restriction fragment-length variants by restriction enzyme digestion and hybridization with a radiolabeled, 1-kb mouse DGAT cDNA fragment as described in Warden, supra. Filters were washed in 1.0 x SSC/0.1% SDS, at 50°C, for 20 min. Autoradiograms were exposed for 3 days at - 70°C. Linkage to previously typed chromosomal markers was detected by using Map Manager v.2.6.5, and loci were ordered by minimizing the number of recombination events between DGAT and the markers. See Manly, Mamm. Genome (1993)4:303-313. The mouse homolog for the DGAT gene (*DGAT*) was mapped to a region of chromosome 15 that exhibits homology with human chromosome 8. The mouse DGAT gene was found to be colocalized with quantitative trait loci for plasma levels of triacylglycerol-rich lipoproteins.
**II. Identification of DGAT cDNA from Arabidopsis thaliana.** The plant (A.thaliana) DGAT gene (#AA042298) (SEQ ID NO:04) was identified from BLAST searches of the EST database using mouse DGAT sequences as a probe. The plant DGAT EST protein sequences encoded by plant DGAT genes are 40-50% identical to mammalian DGAT enzymes. Furthermore, the plant DGAT sequences are more closely related to other mammalian DGAT sequences than to ACAT protein sequences.

### III. Transgenic Animal Studies

### A. Preparation and Characterization of DGAT knockout mice.

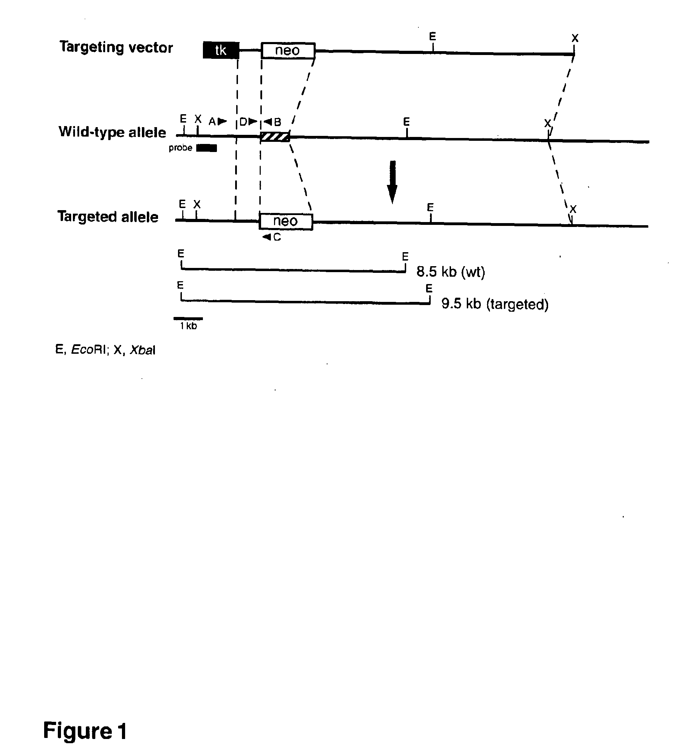

DGAT knockout mice were generated using standard techniques of gene targeting. A mouse P1 clone containing the mouse DGAT gene was isolated from a genomic 129/Sv library. Short and long arms of homologous sequences were amplified by PCR from this clone and subcloned intopNTKLoxP to generate a gene targeting vector. The vector contained a neomycin resistance gene for positive selection and a thymidine kinase gene for negative selection. Upon homologous recombination, the vector was designed to interrupt the DGAT coding sequences at amino acid 360 of the 498-amino acid murine protein. The entire C-terminus, including a highly conserved region common to all ACAT gene family members is deleted. The gene targeting vector was electroporated into RF8 embryonic stem cells by electroporation, and several targeted clones were identified by Southern blotting (targeting frequency of∼1 in 300). The above strategy is depicted in Fig. 1.

One of these targeted clones was injected into C57BL/6 blastocysts and chimeras were generated; male chimeras subsequently passed the DGAT knockout mutation through the germline to their offspring. The resultant mice, which were heterozygous for the DGAT gene disruption, were intercrossed to generate mice that were homozygotes.

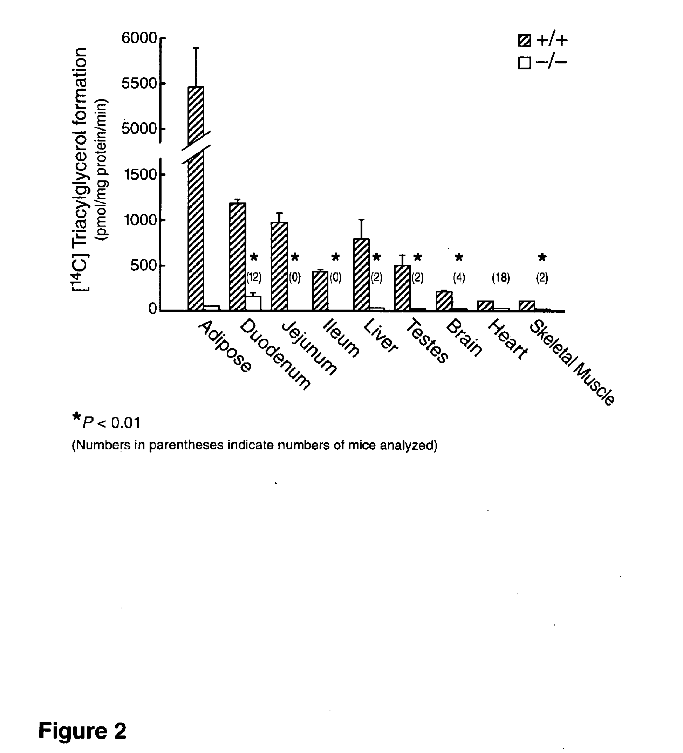

Inactivation of the DGAT gene in the homozygote knockouts was verified by examining DGAT mRNA which was found to be absent in the knockout mice. In activation of the DGAT gene was also verified by studying DGAT activity in tissues using an assay that measures the incorporation of [14C]oleoyl CoA into triglycerides. The results from the activity assays are provided in Fig. 2, and show that DGAT activity is virtually gone from every nearly every tissue tested.

### B. Phenotypic Studies

### 1. General

The resultant DGAT knockout mice were viable, ostensibly healthy, and fertile.

### 2. Fat Analysis

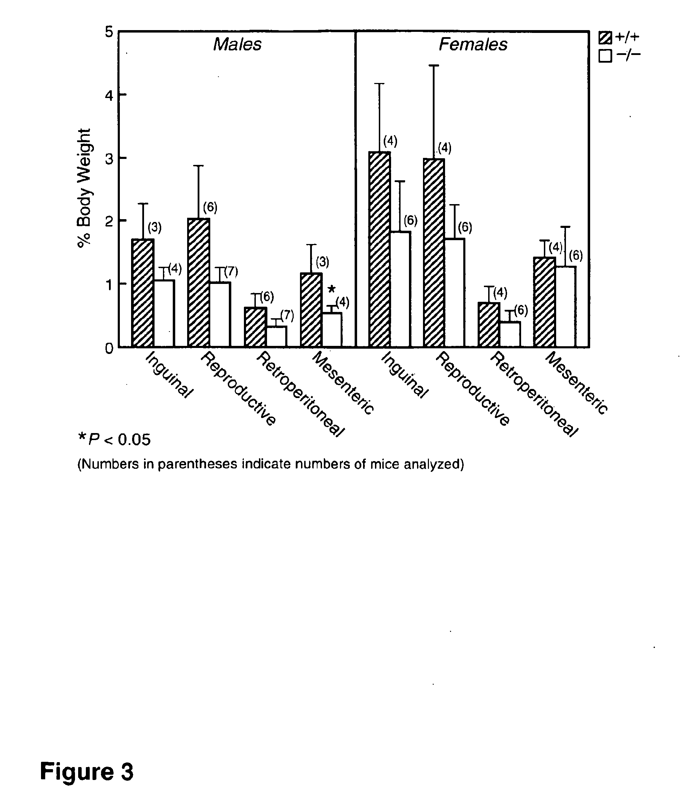

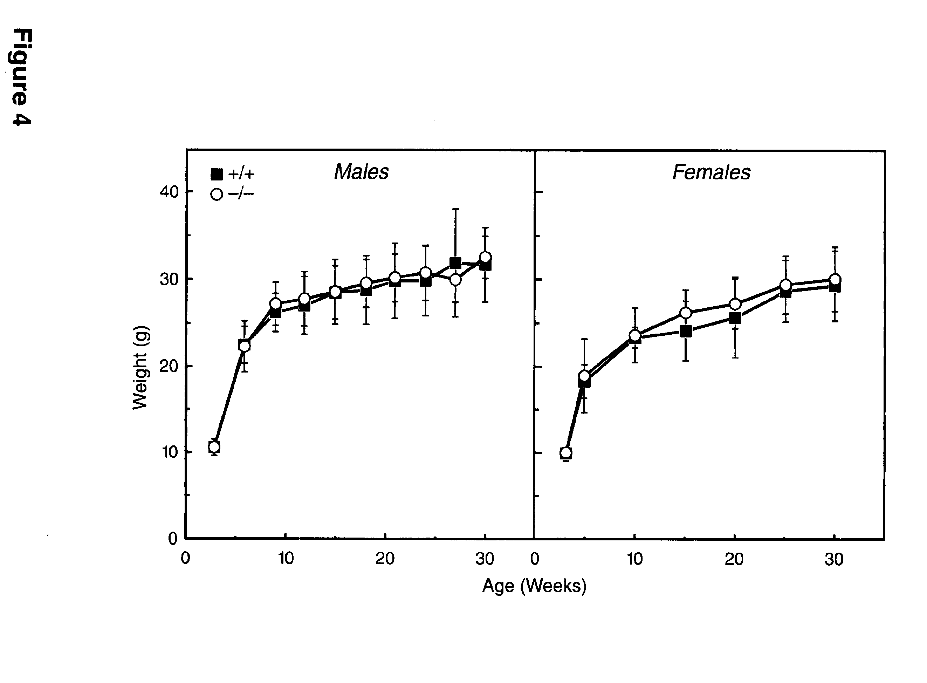

DGAT knockout mice were leaner than their wild type counterparts. This appeared to be true more for males than for females. An analysis of fat pads of male mice fed a chow diet for several months is provided in Fig. 3. Analysis of the fat pads from different regions shows that fat pad weights trend downward in all cases except for the mesenteric fat in females. These data indicate that although chow fed knockout mice weighed the same as chow fed wild type mice, See Fig. 4, the knockout mice are leaner and have reduced adipose tissue. The reproductive and retroperitoneal fat pad sizes are clearly reduced in size (by 50% or more) as compared with those from wild type male mice (Fig. 3). The reduction in fat pads in females appears be not as prominent. Mesenteric fat (visceral fat located in the abdominal cavity) is also clearly reduced in male mice. This is of interest as high levels of mesenteric fat in humans have been associated with insulin resistance, and related syndromes that carry increased risk of heart disease. As such, inhibitors of DGAT activity are useful in the treatment of disease conditions such as insulin resistance and related syndromes, such as those that increased risk of heart disease.

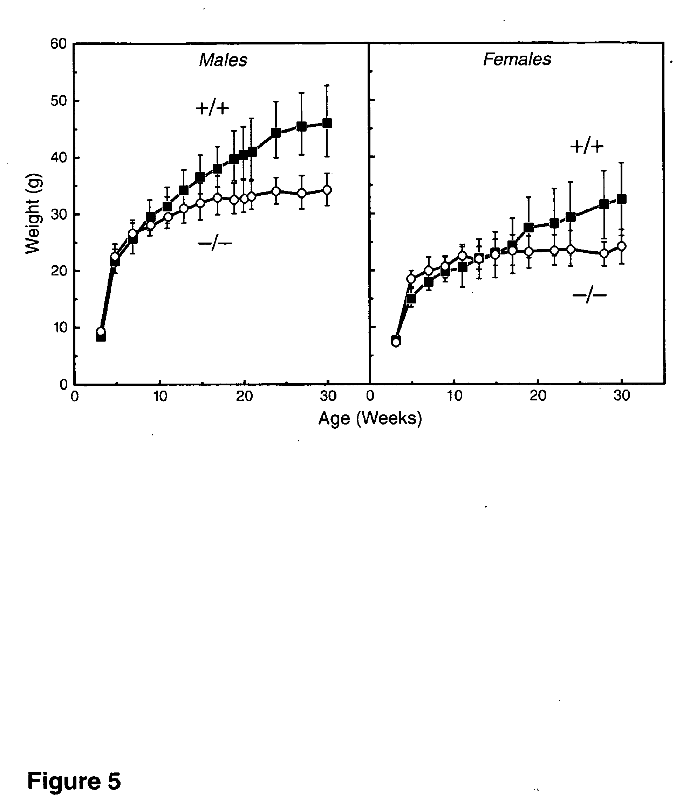

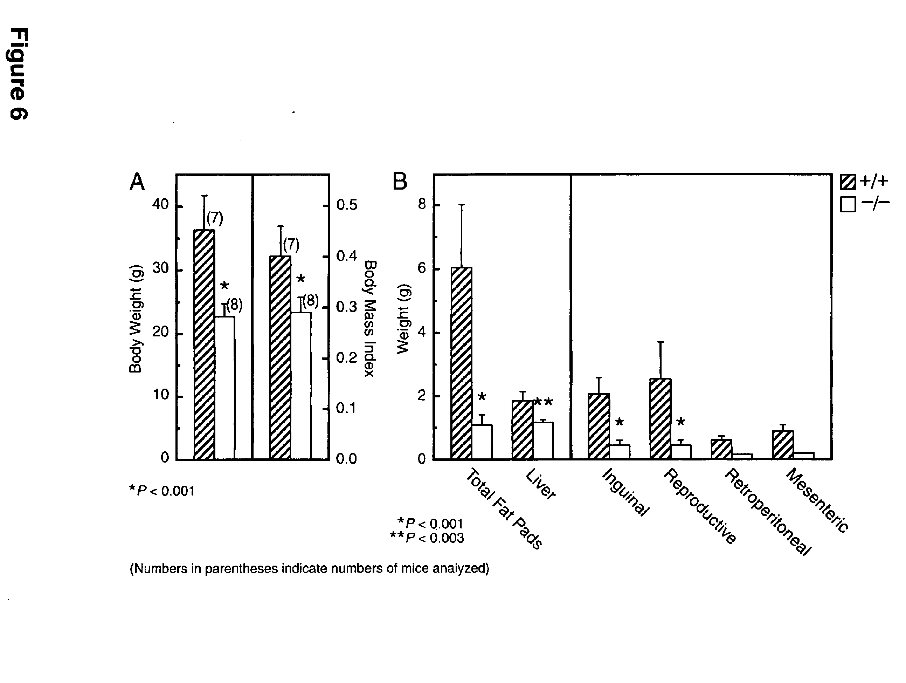

When the knockout mice were fed a diet rich in fat (42% of calories as fat), they were protected from developing dietary induced obesity, as shown in Fig.5. Wherease wild-type mice increased their weight to 45-50 grams on this diet, the knockout mice were protected from developing obesity and, in fact, maintained their body weight in the range of chow fed animals. This protection from dietary induced obesity occurs both in male and female knockout mice. This reduction in body weight, as compared with wild type mice, is primarily due to decreases in adipose tissue, as shown in Figs. 6A and 6B. Fig 6A shows that body weight is significantly decreased in DGAT knockout female mice fed a high-fat diet. In addition, body mass index is significantly decreased. Fig. 6B illustrates that the decrease in weight occurs primarily from a loss of fat-pad mass, although there is a small but significant loss of liver mass as well. Thus, the protection from dietary induced obesity in the DGAT knockout mice results from lower amounts of adipose tissue. As such, inhibitors of DGAT activity are useful in the treatment of dietary induced obesity and disease conditions associated therewith.

Histologic sections of adipose tissue from both wild-type and knockout mice fed a chow diet were also examined and compared. The adipocytes in the fat pads of the knockout mice were reduced in diameter as compared to those observed from the wild-type mice, indicating that the DGAT mutation leads to smaller fat cells.

DGAT knockout mice were also crossed into mice carrying the Agouti mutation, a dominant mutation in mice that leads to yellow fur and obesity from overeating. Mice carrying the Agouti mutation and wild type at the DGAT locus were larger than mice carrying the Agouti mutation and -/- at the DGAT locus. These results indicated that the DGAT knockout mice are protected from genetic forms of obesity. As such, inhibitors of DGAT activity are useful in the treatment of genetically induced obesity.

The livers of both wild type and DGAT knockout mice fed a high fat diet were also compared. While the wild-type mice developed large lipid droplets in their livers, the DGAT mice showed much less fat accumulation in the liver, indicating that the DGAT knockout mice were protected from the development of fatty liver.

In sum, the above results demonstrate that inhibitors of DGAT lower fat stores and protect against diet-induced obesity. As mice completely lacking DGAT are healthy, such inhibitors are of low toxicity.

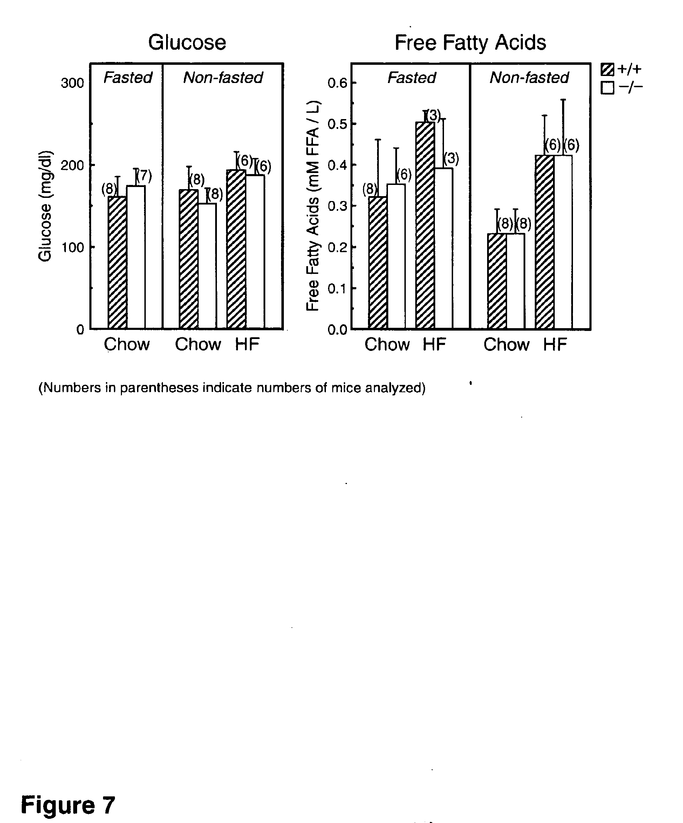

Fig. 7 illustrates that the DGAT knockout mutation has no apparent influence on plasma glucose or free-fatty acid levels. In addition, no differences were detected in insulin levels in the knockout mice as compared to wild-type controls. These results indicate that the DGAT knockout mutation does not affect glucose metabolism in an adverse way. This data further indicates that reduction or elimination of DGAT activity reduces adipose tissue and improves glucose metabolism by causing diminished insulin resistance. As such, inhibitors of DGAT find use in therapy to treat insulin resistance or diabetes associated with obesity.

### 3. Chylomicron Formation

It was also observed that chylomicron formation by the small intestine is impaired in the DGAT knockout mice. Chylomicrons were examined in the bloodstream by agarose gel electrophoresis after administering a corn oil bolus to the mice. It was found that 3 knockout mice had significantly less chylomicrons than the wild type mice. These results indicate that DGAT plays a role in intestinal lipid absorption and that DGAT inhibitors may have a role in diminishing intestinal fat absorption. In sum, the above results indicate that DGAT inhibitors find use as therapeutic agents to lower body fat content.

### 4. Fur Patterns

2 sets of DGAT +/+ and -/- male mice at age about 3 months (young adult), 3-5 in each group per set, were studied. The -/- mice were confirmed to be so by Southern blot analysis. Upon visual examination, the -/- mice appeared normal except that the -/- mice in the second group had some patchy fur loss on the back, mostly in the intrascapular region, the degree of which was, however, highly individual. The mice lacked oils created in the skin that coat the hair, which was due to DGAT deficiency in the sebaceous glands, which are atrophic in the knockout mice. As such, DGAT inhibitors can be used to inhibit skin oil production and are therefore useful in the treatment of acne.

### 5. Wound Healing

Older females (∼8 months old) 2 animals in each group (+/+ and -/-) were examined. One animal had been severely bitten so sacrifice was delayed until the mouse healed (injury results in changes in triglyceride metabolism). She appeared to heal normally suggesting that the DGAT gene that was inactivated in the -/- mice was not essential to the healing process.

### 6. Lactation

The other female gave birth to a healthy appearing litter. However, the pups died soon after birth because they could not suckle (as shown by lack of milk in the pups' stomachs/intestines). On inspection, the mother appeared unable to produce milk. This suggests either that DDGAT is required for mammary gland differentiation, or because the mammary gland resides in a fat pad, DGAT deficiency results in a decreased fat pad that is insufficient to support mammary gland differentiation/milk production. The data also indicate that mutations in the DGAT gene is involved in breast cancer development. As such, this gene might be a target for diagnosis and/or treatment.

### 7. Aging Phenotype

The phenotype of the female mice, determined 5 weeks after the second animal gave birth (to allow recovery; triglyceride metabolism is altered in pregnancy and the post partum period) was similar to that of the males in the second group, i.e. fur loss in patches, especially in the intrascapular region. Again, very little intrabdominal fat, very little subcutaneous fat and decreased retroperitoneal fat pads were observed. Body weight was 12% lower in the -/- female mice with no changes in serum cholesterol or glucose levels, but with about a 50% decrease in triglycerides. These data suggest that the animals become more affected with age. This observation is likely due to a cumulative effect of decreased fat absorption throughout adult life due to low DGAT activity in the duodenum/jejunum; however, this may also be coupled to decreased ability to store fat in adipose tissue due to adipocyte DGAT-1 deficiency.

### IV. Existence of DGAT-2

A possible decrease in fat absorption raised the question of whether the -/mice were essential fatty acid deficient (EFAD) (which could also explain the hair loss and inability to lactate, see below). Fatty acid content of the serum (plasma) from these sets of mice was determined and no evidence for EFAD was found, suggesting that, at least in these generations of mice and at these ages, EFAD does not play a role. DGAT activity in the -/- livers was decreased to about 20% of the +/+ values, p<0.0001. No statistically significant changes were seen in monoacylglycerol or diacylglycerol (DAG) synthesis or in cholesterol ester synthesis. Triglyceride mass in liver total cell membranes was decreased about 70%, p<0.01 with no apparent change in DAG mass. In addition, as shown above, the plasma lipid levels are normal in DGAT knockout mice. Fasting serum triglyceride levels are also normal in DGAT mice consuming either a chow or high fat diet. These data demonstrate the existence of a second DGAT gene, DGAT-2, which is a target for the treatment of high plasma triglyceride levels. Accordingly, DGAT-2 inhibitors find use in the treatment of high plasma triglyceride levels and disease conditions associated therewith.

It is apparent from the above results and discussion that polynucleotides encoding both animal and plant DGAT enzymes, as well as novel polypeptides encoded thereby, are provided. The subject invention is important for both research and therapeutic applications. Using the DGAT probes of the subject invention, the role of DGAT and its regulation in a number of physiological processes can be studied in vivo. The subject invention also provides for important new ways of treating diseases associated with DGAT, such as hypertriglycemia and obesity, as well as in the production of tryglycerides. The citation of any publication is for its disclosure prior to the filing date and should not be construed as an admission that the present invention is not entitled to antedate such publication by virtue of prior invention.

## Claims

1. A non-human transgenic mammal, wherein the non-human transgenic mammal comprises a diacylglycerol O-acyltransferase (DGAT) transgene encoding an enzymatically active DGAT polypeptide, and wherein the DGAT transgene comprises a nucleotide sequence having at least 75% nucleotide sequence identity to the nucleotide sequence set forth in SEQ ID NO:3.

2. The mammal according to claim 1, wherein the transgene is a DGAT transgene comprising a nucleotide sequence having at least 90% nucleotide sequence identity to the nucleotide sequence set forth in SEQ ID NO:3.

3. The mammal according to claim 1, wherein the transgene is a DGAT transgene comprising a nucleotide sequence having at least 95% nucleotide sequence identity to the nucleotide sequence set forth in SEQ ID NO:3.

4. The mammal according to claim 1, wherein said DGAT transgene comprises a non-endogenous nucleic acid.

5. A non-human transgenic knock-out mammal, wherein the non-human transgenic knock-out mammal comprises a disrupted endogenous diacyglycerol O-acyltransferase (DGAT) locus, and wherein said knock-out mammal has reduced adipose tissue compared to a non-transgenic mammal of the same species.

6. The mammal according to any one of claims 1 to 5, wherein said mammal is a rat or a mouse.

7. The knock-out mammal according to claim 5 wherein the knock-out mammal is leaner than a non-transgenic mammal of the same species.

8. The knock-out mammal according to claim 5 wherein the fat cells in said knock-out mammal are smaller than fat cells in a non-transgenic mammal of the same species.

9. A transgenic cell comprising a diacylglycerol O- acyltransferase (DGAT) transgene encoding an enzymatically active (DGAT polypeptide, wherein the DGAT transgene comprises a nucleotide sequence having at least75% nucleotide sequence identity to the nucleotide sequence set forth in SEQ ID NO:3.

10. A transgenic cell derived from the non-human transgenic knock-out mammal of claim 5, wherein the transgenic cell comprises a disrupted endogenous diacylglycerol O-acyltransferase (DGAT) locus.

11. A method of screening a test candidate for its ability to modulate diacylglycerol O-acyltransferase (DGAT) activity, said method comprising:
contacting a mammal according to any one of claims 1-4 or a cell according to claim 9 with said test candidate; and
determining the effect of said test candidate on DGAT activity in said mammal or cell.

## Patentansprüche

1. Nicht-menschliches transgenes Säugetier, worin das nicht-menschliche transgene Säugetier ein Diacylglycerin-O-acyltransferase- (DGAT-) Transgen umfasst, das für ein enzymatisch aktives DGAT-Polypeptid kodiert, und worin das DGAT-Transgen eine Nucleotidsequenz mit zumindest 75 % Nucleotidsequenzidentität mit der in Seq.-ID Nr. 3 dargelegten Nucleotidsequenz umfasst.

2. Säugetier nach Anspruch 1, worin das Transgen ein DGAT-Transgen ist, das eine Nucleotidsequenz mit zumindest 90 % Nucleotidsequenzidentität mit der in Seq.-ID Nr. 3 dargelegten Nucleotidsequenz umfasst.

3. Säugetier nach Anspruch 1, worin das Transgen ein DGAT-Transgen ist, das eine Nucleotidsequenz mit zumindest 95% Nucleotidsequenzidentität mit der in Seq.-ID Nr. 3 dargelegten Nucleotidsequenz umfasst.

4. Säugetier nach Anspruch 1, worin das DGAT-Transgen eine nicht-endogene Nucleinsäure umfasst.

5. Nicht-menschliches transgenes Knock-out-Säugetier, worin das nicht-menschliche transgene Knock-out-Säugetier einen aufgeschlossenen endogenen Diacylglycerin-O-acyltransferase- (DGAT-) Locus umfasst und worin das Knock-out-Säugetier vermindertes adipöses Gewebe im Vergleich zu einem nicht-transgenen Säugetier derselben Spezies aufweist.

6. Säugetier nach einem der Ansprüche 1 bis 5, worin das Säugetier eine Ratte oder eine Maus ist.

7. Knock-out-Säugetier nach Anspruch 5, worin das Knock-out-Säugetier schlanker ist als ein nicht-transgenes Säugetier derselben Spezies.

8. Knock-out-Säugetier nach Anspruch 5, worin die Fettzellen bei dem Knockout-Säugetier kleiner sind als Fettzellen bei einem nicht-transgenen Säugetier derselben Spezies.

9. Transgene Zelle, umfassend ein Diacylglycerin-O-acyltransferase- (DGAT-) Transgen, das für ein enzymatisch aktives DGAT-Polypeptid kodiert, worin das DGAT-Transgen eine Nucleotidsequenz mit zumindest 75 % Nucleotidsequenzidentität mit der in Seq.-ID Nr. 3 dargelegten Nucleotidsequenz umfasst.

10. Transgene Zelle, die von dem nicht-menschlichen transgenen Knock-out-Säugetier nach Anspruch 5 stammt, worin die transgene Zelle einen aufgeschlossenen endogenen Diacylglycerin-O-acyltransferase- (DGAT-) Locus umfasst.

11. Verfahren zum Screening eines Testkandidaten auf seine Fähigkeit, die Diacylglycerin-O-acyltransferase- (DGAT-) Aktivität zu modulieren, wobei das Verfahren Folgendes umfasst:
das Kontaktieren eines Säugetiers nach einem der Ansprüche 1-4 oder einer Zelle nach Anspruch 9 mit dem Testkandidaten; sowie
das Bestimmen der Wirkung des Testkandidaten auf die DGAT-Aktivität in dem Säugetier oder der Zelle.

## Revendications

1. Mammifère transgénique non humain, où le mammifère transgénique non humain comprend un transgène de diacylglycérol O-acyltransférase (DGAT) codant pour un polypeptide de DGAT enzymatiquement actif, et où le transgène de DGAT comprend une séquence de nucléotides ayant au moins 75% d'identité de séquence des nucléotides avec la séquence de nucléotides indiquée à SEQ ID NO:3.

2. Mammifère selon la revendication 1, où le transgène est un transgène de DGAT comprenant une séquence de nucléotides ayant au moins 90% d'identité de séquence de nucléotides avec la séquence de nucléotide indiquée SEQ ID NO:3.

3. Mammifère selon la revendication 1, où le transgène est un transgène de DGAT comprenant une séquence de nucléotides ayant au moins 95% d'identité de séquence de nucléotides avec la séquence de nucléotides indiquée à SEQ ID NO:3.

4. Mammifère selon la revendication 1, où ledit transgène de DGAT comprend un acide nucléique non endogène.

5. Mammifère knock-out transgénique non humain, où le mammifère knock-out transgénique non humain comprend un locus de diacylglycérol *O*-acyltransférase (DGAT) endogène interrompu, et où ledit mammifère knock-out a un tissu adipeux réduit en comparaison avec un mammifère non transgénique de la même espèce.

6. Mammifère selon l'une quelconque des revendications 1 à 5, où ledit mammifère est un rat ou une souris.

7. Mammifère knock-out selon la revendication 5 où le mammifère knock-out est plus maigre qu'un mammifère non transgénique de la même espèce.

8. Mammifère knock-out selon la revendication 5 où les cellules grasses dans ledit mammifère knock-out sont plus petites que les cellules grasses dans un mammifère non transgénique de la même espèce.

9. Cellule transgénique comprenant un transgène de diacylglycérol O-acyltransférase (DGAT) codant pour un polypeptide de DGAT enzymatiquement actif, où le transgène de DGAT comprend une séquence de nucléotides ayant au moins 75% d'identité de séquence de nucléotides avec la séquence de nucléotides indiquée à SEQ ID NO:3.

10. Cellule transgénique dérivée du mammifère knockout transgénique non humain de la revendication 5, où la cellule transgénique comprend un locus de diacylglycérol O-acyltransférase (DGAT) interrompu endogène.

11. Méthode de criblage d'un candidat de test pour son aptitude à moduler l'activité de diacylglycérol O-acyltransférase (DGAT), ladite méthode consistant à:
mettre en contact un mammifère selon l'une quelconque des revendications 1-4 ou une cellule selon la revendication 9 avec ledit candidat de test; et
déterminer l'effet dudit candidat de test sur l'activité de DGAT dudit mammifère ou de ladite cellule.


### SEQUENCE LISTING

<110> Farese, Robert V.
   Cases, Sylvaine
   Smith, Steven
   Erickson, Sandra
   Sande, Eric
<120> Diacylglycerol O-Acyltransferase
<130> 6510-105US3
<150> 60/107,771
   <151> 1998-11-09
<150> PCT/US98/17883
   <151> 1998-08-28
<150> 09/103,754
   <151> 1998-06-24
<160> 10
<170> FastSEQ for Windows Version 3.0
<210> 1
   <211> 1411
   <212> DNA
   <213> homo sapiens
<400> 1
   ttatttttgg agaacctcat caagtatggc atcctggtgg accccatcca ggtggtttct      60
   ctgttcctga aggatcccta tagctggccc gccccatgcc tggttattgc ggccaatgtt     120
   tttgctgtgg ctgcattcca ggttgagaag cgcctggcgg tgggtgccct gacggagcag     180
   gcgggactgc tgctgcacgt ggccaacctg gccaccattc tgtgtttccc agcggctgtg     240
   gtcttactgg ttgagtctat cactccagtg ggctccctgc tggcgctgat ggcgcacacc     300
   atcctcttcc tcaagctctt ctcctaccgc gacgtcaact catggtgccg cagggccagg     360
   gccaaggctg cctctgcagg gaagaaggcc agcagtgttg ctgccccgca caccgtgagc     420
   tacccggaca atctgaccta ccgcgatctc tactacttcc tcttcgcccc caccttgtgc     480
   tacgagctca actttccccg ctctccccgc atccggaagc gctttctgct gcgacggatc     540
   cttgagatgc tgttcttcac ccagctccag gtggggctga tccagcagtg gatggtcccc     600
   accatccaga actccatgaa gcccttcaag gacatggact actcacgcat catcgagcgc     660
   ctcctgaagc tggcggtccc caatcacctc atctggctca tcttcttcta ctggctcttc     720
   cactcctgcc tgaatgccgt ggctgagctc atgcagtttg gagaccggga gttctaccgg     780
   gactggtgga actccgagtc tgtcacctac ttctggcaga actggaacat ccctgtgcac     840
   aagtggtgca tcagacactt ctacaagccc atgcttcgac ggggcagcag caagtggatg     900
   gccaggacag gggtgttcct ggcctcggcc ttcttccacg agtacctggt gagcgtccct     960
   ctgcgaatgt tccgcctctg ggcgttcacg ggcatgatgg ctcagatccc actggcctgg    1020
   ttcgtgggcc gctttttcca gggcaactat ggcaacgcag ctgtgtggct gtcgctcatc    1080
   atcggacagc caatagccgt cctcatgtac gtccacgact actacgtgct caactatgag    1140
   gccccagcgg cagaggcctg agctgcacct gaggggctgg cttctcactg ccacctcaca    1200
   cccgctggca gagcccacct ctcctcctag gcctcgagtt gctggggatg ggcctggctg    1260
   cacagcatcc tcctctggtc ccagggaggc ctctctgccc ctatggggct ctgtcctgca    1320
   cccctcaggg atggcgacag caggccagac acagtctgat gccagctggg agtcttgctg    1380
   accctgcccc gggtccgagg gtgtcaataa a                                    1411
<210> 2
   <211> 261
   <212> DNA
   <213> homo sapiens
<400> 2
   cagaggcctg agctgcacct gaggggctgg cttctcactg ccacctcaca cccgctggca     60
   gagcccacct ctcctcctag gcctcgagtt gctggggatg ggcctggctg cacagcatcc    120
   tcctctggtc ccagggaggc ctctctgccc ctatggggct ctgtcctgca cccctcaggg    180
   atggcgacag caggccagac acagtctgat gccagctggg agtcttgctg accctgcccc    240
   gggtccgagg gtgtcaataa a                                              261
<210> 3
   <211> 1650
   <212> DNA
   <213> mus musculus
<400> 3
   ggatgaatgg aaataagtag aattaggcat acttaggata gggctcaagc cgcggcccgt     60
   gaagattggg ccgcgacgag gtgcgggccg aagccatggg cgaccgcgga ggcgcgggaa    120
   gctctcggcg tcggaggacc ggctcgcggg tttccgtcca gggtggtagt gggcccaagg    180
   tagaagagga cgaggtgcga gacgcggctg tgagccccga cttgggcgcc gggggtgacg    240
   cgccggctcc ggctccggct ccagcccata cccgggacaa agacgggcgg accagcgtgg    300
   gcgacggcta ctgggatctg aggtgccatc gtctgcaaga ttctttgttc agctcagaca    360
   gtggtttcag caattatcgt ggtatcctga attggtgtgt ggtgatgctg atcctgagta    420
   atgcaaggtt atttttagag aaccttatca agtatggcat cctggtggat cctatccagg    480
   tggtgtctct gtttttgaag gacccctaca gctggcctgc cccatgcgtg attattgcat     540
   ccaatatttt tgttgtggct gcatttcaga ttgagaagcg cctggcagtg ggtgccctga    600
   cagagcagat ggggctgctg ctacatgtgg ttaacctggc cacaatcatt tgcttcccag    660
   cagctgtggc cttactggtt gagtctatca ctccagtggg ttccgtgttt gctctggcat    720
   catactccat catgttcctc aagctttatt cctaccggga tgtcaacctg tggtgccgcc    780
   agcgaagggt caaggccaaa gctgtctcta cagggaagaa ggtcagtggg gctgctgccc    840
   agcaagctgt gagctatcca gacaacctga cctaccgaga tctctattac ttcatctttg    900
   ctcctacttt gtgttatgaa ctcaactttc ctcggtcccc cgcaatacga aagcgctttc    960
   tgctacgacg agttcttgag atgctctttt ttacccagct tcaagtgggg ctgatccaac   1020
   agtggatggt ccctactatc cacaactcca tgaagccctt caaggatatg gactattcac   1080
   ggatcattga gcgtctctta aagctggcgg tccccaacca tctgatctgg cttatcttct   1140
   tctattggtt tttccactcc tgtctcaatg ctgtggcaga gcttctgcag tttggagacc   1200
   gcgagttcta cagagattgg tggaatgctg agtctgtcac ctacttttgg cagaactgga   1260
   atatccccgt gcacaagtgg tgcatcagac acttctacaa gcctatgctc agacatggca   1320
   gcagcaaatg ggtggccagg acaggagtat ttttgacctc agccttcttc catgagtacc    1380
   tagtgagcgt tcccctgcgg atgttccgcc tctgggcatt cacagccatg atggctcagg   1440
   tcccactggc ctggattgtg ggccgattct tccaagggaa ctatggcaat gcagctgtgt   1500
   gggtgacact catcattggg caaccggtgg ctgtgctcat gtatgtccac gactactacg   1560
   tgctcaacta cgatgcccca gtgggggtat gagctactgc caaaggccag ccctccctaa   1620
   cctgggcctg gagttctgga ggggttcctg                                    1650
<210> 4
   <211> 629
   <212> DNA
   <213> arabidopsis thaliana
<400> 4
   tgcatgtata cggaagggtt gggtggctcg tcaatttgca aaactggtca tattcaccgg      60
   attcatggga tttataatag aacaatatat aaatcctatt gtcaggaact caaagcatcc     120
   tttgaaaggc gatcttctat atgctattga aagagtgttg aagctttcag ttccaaattt     180
   atatgtgtgg ctctgcatgt tctactgctt cttccacctt tggttaaaca tattggcaga     240
   gcttctctgc ttcggggatc gtgaattcta caaagattgg tggaatgcaa aaagtgtggg     300
   agattactgg gagaatgtgg aatatgcctg tccataaatg ggatgggtcc gacatatata     360
   ccttccccgt gcttgcgcac aaggattacc caaagacacc ccggccatta accattggct     420
   ttcccaagcc ccctggaggc ctttccatgg gccanggacc cggngtnccc tggcnggccc     480
   ttcaaagcaa agggggnttn cctggggnta aagntccang ggcccttggg gcccanccaa    540
   aannttcccc cgggaaaggg ttgcccaccg gggggngaaa aanncccggg ggcaccncgg     600
   aattttggga acccgggggg ggccttttt                                      629
<210> 5
   <211> 386
   <212> PRT
   <213> homo sapiens
<400> 5
   Leu Phe Leu Glu Asn Leu Ile Lys Tyr Gly Ile Leu Val Asp Pro Ile
    1               5                  10                  15
   Gln Val Val Ser Leu Phe Leu Lys Asp Pro Tyr Ser Trp Pro Ala Pro
               20                  25                  30
   Cys Leu Val Ile Ala Ala Asn Val Phe Ala Val Ala Ala Phe Gln Val
           35                  40                  45
   Glu Lys Arg Leu Ala Val Gly Ala Leu Thr Glu Gln Ala Gly Leu Leu
       50                  55                  60
   Leu His Val Ala Asn Leu Ala Thr Ile Leu Cys Phe Pro Ala Ala Val
   65                  70                  75                  80
   Val Leu Leu Val Glu Ser Ile Thr Pro Val Gly Ser Leu Leu Ala Leu
                   85                  90                  95
   Met Ala His Thr Ile Leu Phe Leu Lys Leu Phe Ser Tyr Arg Asp Val
               100                 105                 110
   Asn Ser Trp Cys Arg Arg Ala Arg Ala Lys Ala Ala Ser Ala Gly Lys
           115                 120                 125
   Lys Ala Ser Ser Val Ala Ala Pro His Thr Val Ser Tyr Pro Asp Asn
       130                 135                 140
   Leu Thr Tyr Arg Asp Leu Tyr Tyr Phe Leu Phe Ala Pro Thr Leu Cys
   145                 150                 155                 160
   Tyr Glu Leu Asn Phe Pro Arg Ser Pro Arg Ile Arg Lys Arg Phe Leu
                   165                 170                 175
   Leu Arg Arg Ile Leu Glu Met Leu Phe Phe Thr Gln Leu Gln Val Gly
               180                 185                 190
   Leu Ile Gln Gln Trp Met Val Pro Thr Ile Gln Asn Ser Met Lys Pro
           195                 200                 205
   Phe Lys Asp Met Asp Tyr Ser Arg Ile Ile Glu Arg Leu Leu Lys Leu
       210                 215                 220
   Ala Val Pro Asn His Leu Ile Trp Leu Ile Phe Phe Tyr Trp Leu Phe
   225                 230                 235                 240
   His Ser Cys Leu Asn Ala Val Ala Glu Leu Met Gln Phe Gly Asp Arg
                   245                 250                 255
   Glu Phe Tyr Arg Asp Trp Trp Asn Ser Glu Ser Val Thr Tyr Phe Trp
               260                 265                 270
   Gln Asn Trp Asn Ile Pro Val His Lys Trp Cys Ile Arg His Phe Tyr
           275                 280                 285
   Lys Pro Met Leu Arg Arg Gly Ser Ser Lys Trp Met Ala Arg Thr Gly
       290                 295                 300
   Val Phe Leu Ala Ser Ala Phe Phe His Glu Tyr Leu Val Ser Val Pro
   305                 310                 315                 320
   Leu Arg Met Phe Arg Leu Trp Ala Phe Thr Gly Met Met Ala Gln Ile
                   325                 330                 335
   Pro Leu Ala Trp Phe Val Gly Arg Phe Phe Gln Gly Asn Tyr Gly Asn
               340                 345                 350
   Ala Ala Val Trp Leu Ser Leu Ile Ile Gly Gln Pro Ile Ala Val Leu
           355                 360                 365
   Met Tyr Val His Asp Tyr Tyr Val Leu Asn Tyr Glu Ala Pro Ala Ala
       370                 375                 380
   Glu Ala
   385
<210> 6
   <211> 488
   <212> PRT
   <213> homo sapiens
<400> 6
   Met Gly Asp Arg Gly Ser Ser Arg Arg Arg Arg Thr Gly Ser Arg Pro
    1               5                  10                  15
   Ser Ser His Gly Gly Gly Gly Pro Ala Ala Ala Glu Glu Glu Val Arg
               20                  25                  30
   Asp Ala Ala Ala Gly Pro Asp Val Gly Ala Ala Gly Asp Ala Pro Ala
           35                  40                  45
   Pro Ala Pro Asn Lys Asp Gly Asp Ala Gly Val Gly Ser Gly His Trp
       50                  55                  60
   Glu Leu Arg Cys His Arg Leu Gln Asp Ser Leu Phe Ser Ser Asp Ser
   65                  70                  75                  80
   Gly Phe Ser Asn Tyr Arg Gly Ile Leu Asn Trp Cys Val Val Met Leu
                   85                  90                  95
   Ile Leu Ser Asn Ala Arg Leu Phe Leu Glu Asn Leu Ile Lys Tyr Gly
               100                 105                 110
   Ile Leu Val Asp Pro Ile Gln Val Val Ser Leu Phe Leu Lys Asp Pro
           115                 120                 125
   His Ser Trp Pro Ala Pro Cys Leu Val Ile Ala Ala Asn Val Phe Ala
       130                 135                 140
   Val Ala Ala Phe Gln Val Glu Lys Arg Leu Ala Val Gly Ala Leu Thr
   145                 150                 155                 160
   Glu Gln Ala Gly Leu Leu Leu His Val Ala Asn Leu Ala Thr Ile Leu
                   165                 170                 175
   Cys Phe Pro Ala Ala Val Val Leu Leu Val Glu Ser Ile Thr Pro Val
               180                 185                 190
   Gly Ser Leu Leu Ala Leu Met Ala His Thr Ile Leu Phe Leu Lys Leu
           195                 200                 205
   Phe Ser Tyr Arg Asp Val Asn Ser Trp Cys Arg Arg Ala Arg Ala Lys
       210                 215                 220
   Ala Ala Ser Ala Gly Lys Lys Ala Ser Ser Ala Ala Ala Pro His Thr
   225                 230                 235                 240
   Val Ser Tyr Pro Asp Asn Leu Thr Tyr Arg Asp Leu Tyr Tyr Phe Leu
                   245                 250                 255
   Phe Ala Pro Thr Leu Cys Tyr Glu Leu Asn Phe Pro Arg Ser Pro Arg
               260                 265                 270
   Ile Arg Lys Arg Phe Leu Leu Arg Arg Ile Leu Glu Met Leu Phe Phe
           275                 280                 285
   Thr Gln Leu Gln Val Gly Leu Ile Gln Gln Trp Met Val Pro Thr Ile
       290                 295                 300
   Gln Asn Ser Met Lys Pro Phe Lys Asp Met Asp Tyr Ser Arg Ile Ile
   305                 310                 315                 320
   Glu Arg Leu Leu Lys Leu Ala Val Pro Asn His Leu Ile Trp Leu Ile
                   325                 330                 335
   Phe Phe Tyr Trp Leu Phe His Ser Cys Leu Asn Ala Val Ala Glu Leu
               340                 345                 350
   Met Gln Phe Gly Asp Arg Glu Phe Tyr Arg Asp Trp Trp Asn Ser Glu
           355                 360                 365
   Ser Val Thr Tyr Phe Trp Gln Asn Trp Asn Ile Pro Val His Lys Trp
       370                 375                 380
   Cys Ile Arg His Phe Tyr Lys Pro Met Leu Arg Arg Gly Ser Ser Lys
   385                 390                 395                 400
   Trp Met Ala Arg Thr Gly Val Phe Leu Ala Ser Ala Phe Phe His Glu
                   405                 410                 415
   Tyr Leu Val Ser Val Pro Leu Arg Met Phe Arg Leu Trp Ala Phe Thr
               420                 425                 430
   Gly Met Met Ala Gln Ile Pro Leu Ala Trp Phe Val Gly Arg Phe Phe
           435                 440                 445
   Gln Gly Asn Tyr Gly Asn Ala Ala Val Trp Leu Ser Leu Ile Ile Gly
       450                 455                 460
   Gln Pro Ile Ala Val Leu Met Tyr Val His Asp Tyr Tyr Val Leu Asn
   465                 470                 475                 480
   Tyr Glu Ala Pro Ala Ala Glu Ala
                   485
<210> 7
   <211> 498
   <212> PRT
   <213> mus musculus
<400> 7
   Met Gly Asp Arg Gly Gly Ala Gly Ser Ser Arg Arg Arg Arg Thr Gly
    1               5                  10                  15
   Ser Arg Val Ser Val Gln Gly Gly Ser Gly Pro Lys Val Glu Glu Asp
               20                  25                  30
   Glu Val Arg Asp Ala Ala Val Ser Pro Asp Leu Gly Ala Gly Gly Asp
           35                  40                  45
   Ala Pro Ala Pro Ala Pro Ala Pro Ala His Thr Arg Asp Lys Asp Gly
       50                  55                  60
   Arg Thr Ser Val Gly Asp Gly Tyr Trp Asp Leu Arg Cys His Arg Leu
   65                  70                  75                  80
   Gln Asp Ser Leu Phe Ser Ser Asp Ser Gly Phe Ser Asn Tyr Arg Gly
                   85                  90                  95
   Ile Leu Asn Trp Cys Val Val Met Leu Ile Leu Ser Asn Ala Arg Leu
               100                 105                 110
   Phe Leu Glu Asn Leu Ile Lys Tyr Gly Ile Leu Val Asp Pro Ile Gln
           115                 120                 125
   Val Val Ser Leu Phe Leu Lys Asp Pro Tyr Ser Trp Pro Ala Pro Cys
       130                 135                 140
   Val Ile Ile Ala Ser Asn Ile Phe Val Val Ala Ala Phe Gln Ile Glu
   145                 150                 155                 160
   Lys Arg Leu Ala Val Gly Ala Leu Thr Glu Gln Met Gly Leu Leu Leu
                   165                 170                 175
   His Val Val Asn Leu Ala Thr Ile Ile Cys Phe Pro Ala Ala Val Ala
               180                 185                 190
   Leu Leu Val Glu Ser Ile Thr Pro Val Gly Ser Val Phe Ala Leu Ala
   195 200 205
   Ser Tyr Ser Ile Met Phe Leu Lys Leu Tyr Ser Tyr Arg Asp Val Asn
   210 215 220
   Leu Trp Cys Arg Gln Arg Arg Val Lys Ala Lys Ala Val Ser Thr Gly
   225 230 235 240
   Lys Lys Val Ser Gly Ala Ala Ala Gln Gln Ala Val Ser Tyr Pro Asp
   245 250 255
   Asn Leu Thr Tyr Arg Asp Leu Tyr Tyr Phe Ile Phe Ala Pro Thr Leu
   260 265 270
   Cys Tyr Glu Leu Asn Phe Pro Arg Ser Pro Arg Ile Arg Lys Arg Phe
   275 280 285
   Leu Leu Arg Arg Val Leu Glu Met Leu Phe Phe Thr Gln Leu Gln Val
   290 295 300
   Gly Leu Ile Gln Gln Trp Met Val Pro Thr Ile His Asn Ser Met Lys
   305 310 315 320
   Pro Phe Lys Asp Met Asp Tyr Ser Arg Ile Ile Glu Arg Leu Leu Lys
   325 330 335
   Leu Ala Val Pro Asn His Leu Ile Trp Leu Ile Phe Phe Tyr Trp Phe
   340 345 350
   Phe His Ser Cys Leu Asn Ala Val Ala Glu Leu Leu Gln Phe Gly Asp
   355 360 365
   Arg Glu Phe Tyr Arg Asp Trp Trp Asn Ala Glu Ser Val Thr Tyr Phe
   370 375 380
   Trp Gln Asn Trp Asn Ile Pro Val His Lys Trp Cys Ile Arg His Phe
   385 390 395 400
   Tyr Lys Pro Met Leu Arg His Gly Ser Ser Lys Trp Val Ala Arg Thr
   405 410 415
   Gly Val Phe Leu Thr Ser Ala Phe Phe His Glu Tyr Leu Val Ser Val
   420 425 430
   Pro Leu Arg Met Phe Arg Leu Trp Ala Phe Thr Ala Met Met Ala Gln
   435 440 445
   Val Pro Leu Ala Trp Ile Val Gly Arg Phe Phe Gln Gly Asn Tyr Gly
   450 455 460
   Asn Ala Ala Val Trp Val Thr Leu Ile Ile Gly Gln Pro Val Ala Val
   465 470 475 480
   Leu Met Tyr Val His Asp Tyr Tyr Val Leu Asn Tyr Asp Ala Pro Val
   485 490 495
   Gly Val
<210> 8
   <211> 10
   <212> PRT
   <213> Artificial Sequence
<220>
   <223> synthetic peptide-FLAG epitope
<400> 8
   Met Gly Asp Tyr Lys Asp Asp Asp Asp Gly
   1 5 10
<210> 9
   <211> 1650
   <212> DNA
<213> mus musculus
<400> 9
<210> 10
   <211> 498
   <212> PRT
   <213> mus musculus
<400> 10
   Met Gly Asp Arg Gly Gly Ala Gly Ser Ser Arg Arg Arg Arg Thr Gly
    1               5                  10                  15
   Ser Arg Val Ser Val Gln Gly Gly Ser Gly Pro Lys Val Glu Glu Asp
               20                  25                  30
   Glu Val Arg Asp Ala Ala Val Ser Pro Asp Leu Gly Ala Gly Gly Asp
           35                  40                  45
   Ala Pro Ala Pro Ala Pro Ala Pro Ala His Thr Arg Asp Lys Asp Gly
       50                  55                  60
   Arg Thr Ser Val Gly Asp Gly Tyr Trp Asp Leu Arg Cys His Arg Leu
   65                  70                  75                  80
   Gln Asp Ser Leu Phe Ser Ser Asp Ser Gly Phe Ser Asn Tyr Arg Gly
                   85                  90                  95
   Ile Leu Asn Trp Cys Val Val Met Leu Ile Leu Ser Asn Ala Arg Leu
               100                 105                 110
   Phe Leu Glu Asn Leu Ile Lys Tyr Gly Ile Leu Val Asp Pro Ile Gln
           115                 120                 125
   Val Val Ser Leu Phe Leu Lys Asp Pro Tyr Ser Trp Pro Ala Pro Cys
       130                 135                 140
   Val Ile Ile Ala Ser Asn Ile Phe Val Val Ala Ala Phe Gln Ile Glu
   145                 150                 155                 160
   Lys Arg Leu Ala Val Gly Ala Leu Thr Glu Gln Met Gly Leu Leu Leu
                   165                 170                 175
   His Val Val Asn Leu Ala Thr Ile Ile Cys Phe Pro Ala Ala Val Ala
               180                 185                 190
   Leu Leu Val Glu Ser Ile Thr Pro Val Gly Ser Val Phe Ala Leu Ala
           195                 200                 205
   Ser Tyr Ser Ile Met Phe Leu Lys Leu Tyr Ser Tyr Arg Asp Val Asn
       210                 215                 220
   Leu Trp Cys Arg Gln Arg Arg Val Lys Ala Lys Ala Val Ser Thr Gly
   225                 230                 235                 240
   Lys Lys Val Ser Gly Ala Ala Ala Gln Gln Ala Val Ser Tyr Pro Asp
                   245                 250                 255
   Asn Leu Thr Tyr Arg Asp Leu Tyr Tyr Phe Ile Phe Ala Pro Thr Leu
               260                 265                 270
   Cys Tyr Glu Leu Asn Phe Pro Arg Ser Pro Arg Ile Arg Lys Arg Phe
           275                 280                 285
   Leu Leu Arg Arg Val Leu Glu Met Leu Phe Phe Thr Gln Leu Gln Val
       290                 295                 300
   Gly Leu Ile Gln Gln Trp Met Val Pro Thr Ile His Asn Ser Met Lys
   305                 310                 315                 320
   Pro Phe Lys Asp Met Asp Tyr Ser Arg Ile Ile Glu Arg Leu Leu Lys
                   325                 330                 335
   Leu Ala Val Pro Asn His Leu Ile Trp Leu Ile Phe Phe Tyr Trp Phe
               340                 345                 350
   Phe His Ser Cys Leu Asn Ala Val Ala Glu Leu Leu Gln Phe Gly Asp
           355                 360                 365
   Arg Glu Phe Tyr Arg Asp Trp Trp Asn Ala Glu Ser Val Thr Tyr Phe
       370                 375                 380
   Trp Gln Asn Trp Asn Ile Pro Val His Lys Trp Cys Ile Arg His Phe
   385                 390                 395                 400
   Tyr Lys Pro Met Leu Arg His Gly Ser Ser Lys Trp Val Ala Arg Thr
                   405                 410                 415
   Gly Val Phe Leu Thr Ser Ala Phe Phe His Glu Tyr Leu Val Ser Val
               420                 425                 430
   Pro Leu Arg Met Phe Arg Leu Trp Ala Phe Thr Ala Met Met Ala Gln
           435                 440                 445
   Val Pro Leu Ala Trp Ile Val Gly Arg Phe Phe Gln Gly Asn Tyr Gly
       450                 455                 460
   Asn Ala Ala Val Trp Val Thr Leu Ile Ile Gly Gln Pro Val Ala Val
   465                 470                 475                 480
   Leu Met Tyr Val His Asp Tyr Tyr Val Leu Asn Tyr Asp Ala Pro Val
                   485                 490                 495
   Gly Val